# EUROPEAN PATENT SPECIFICATION

(11) **EP 0 826 386 B1**
(45) Date of publication and mention of the grant of the patent: **26.11.2003**
(21) Application number: 97118730.7
(22) Date of filing: 24.08.1992
(51) Int. Cl.: A61M 15/00, G01F 11/24

(54) **Powder dispenser**
Pulverspender
Distributeur de poudre

(30) Priority: 26.08.1991 US 749912; 06.01.1992 US 817331
(43) Date of publication of application: 04.03.1998
(62) Divisional of application: 92919070.0
(73) Proprietor: MINNESOTA MINING AND MANUFACTURING COMPANY, St. Paul, Minnesota 55133-3427 (US)
(72) Inventor: Whaley, Ralph D., c/o Minnesota Mining and, St. Paul, Minnesota 55133-3427 (US); Shinnick, Mark S., c/o Minnesota Mining and, St. Paul, Minnesota 55133-3427 (US); Thiel, Charles G., c/o Minnesota Mining and, St. Paul, Minnesota 55133-3427 (US); Kriegl, DonGene, c/o Minnesota Mining and, St. Paul, Minnesota 55133-3427 (US); Reeder, Thomas W., c/o Minnesota Mining and, St. Paul, Minnesota 55133-3427 (US); Pattock, Brian M., c/o Minnesota Mining and, St. Paul, Minnesota 55133-3427 (US); Mattila, Robert J., c/o Minnesota Mining and, St. Paul, Minnesota 55133-3427 (US); Turgeon, Robert J., c/o Minnesota Mining and, St. Paul, Minnesota 55133-3427 (US)
(74) Representative: VOSSIUS & PARTNER

(56) References cited:
- EP-A- 0 254 391
- EP-A- 0 424 790
- WO-A-90/07351
- WO-A-92/00771
- WO-A-92/04068
- GB-A- 2 144 997
- US-A- 4 524 769

## Description

The present invention relates generally to devices which facilitate the inhalation of powdered medicament.

Dose uniformity or accuracy is a problem associated with known dispensers which dispense powdered medicament. Generally, powdered medicaments tend to "pack" into unduly large agglomerates, particularly when the size of the particles is in the order of a few microns. If the medicament is delivered to a user in unduly large agglomerates, the large agglomerates tend to impact the tissue at the back of the user's throat and thus the medicament is prevented from reaching the user's lungs. Such impact also sometimes causes an uncomfortable "gag" reflex or coughing. Further, it is desirable to completely disperse the entire dose delivered to a user to maximize the respirable fraction and efficacy of the medicament delivered to the user and to avoid the attendant problems associated with large agglomerates of powder.

Rosskamp et al. U.S. Patent 4,046,146; U.S. Patent 4,811,731 to Newell et al.; U.S. Patent 2,587,215 to Priestly and U.S. Patent 4,524,769 to Wetterlin all describe inhalators that are powered by the user's inhalation airflow. One problem associated with user powered dispensers is that the user's capacity to generate an effective airflow may be adversely affected by an ailment such as bronchial asthma. It is believed that the powdered medicament dispensers which rely upon user generated airflow tend to be inefficient, particularly when a user's capacity to generate an inhalation airflow is adversely affected by an ailment. Another problem associated with user powered dispensers is that different persons possess widely varying abilities to generate inhalation airflows. Thus, requiring a user to generate a considerable airflow may cause discomfort for some users.

Wetterlin 4,524,769 describes a device similar to a TURBOHALER T.M. dispenser which is generally available from Aktiebolaget Draco, of Lund, Sweden and is believed to be on sale in Europe. That device includes a perforated member with a plurality of perforations for each dosage of medicament. The TURBOHALER T.M. dispenser is a user powered inhalation device and is believed to suffer from the problems associated with user powered medicament dispensers mentioned above.

The art also has devices which facilitate the inhalation of powdered medicaments by the use of propellants to dispense and disperse the medicament. Dispensers which utilize a propellant are disclosed in PCT application Number WO 90/07351 and Wetterlin U.S. Patents No. 4,534,345 and 4,667,668. GB-A- 2 144 997 describes the use in powder inhalators of a perforated membrane as metering and holding means for a predetermined amount of solid micronised pharmacologically active compounds. In particular, the document describes to provide
(a) a propellant container and a propellant dispensing unit; and
(b) a dosing unit for dosing the pharmacologically active compound, characterized in that the dosing unit for dosing the pharmacologically active compound comprises a storage chamber for the active compound directly connected to a dosing unit, said dosing unit comprising a perforated membrane, displaceably arranged in relation to the storage chamber, a holder for the perforated membrane and means for displacing the membrane, whereby the membrane is displaceably arranged between a first position where active compound is introduced into the perforations in at least a part of the area of the membrane and a second position where that part of the membrane is inserted in a propellant passage leading from the propellant dispensing unit.
Other prior art dispensers use chlorofluorocarbon to deliver a pharmaceutically active compound. In these types of dispensers, the medicament is typically suspended within the chlorofluorocarbon propellant. While such dispensers are somewhat successful at delivering medicament to a user, it is not always desirable to use chlorofluorocarbon propellants for reasons including cost. Also, dispensers such as those mentioned above are believed to be complex to construct, inefficient or difficult to use.

It is the object of the present invention to provide an improved dry powder medicament dispenser and the use thereof. This object is achieved with the respective claims.

The present invention provides a simplified dry powder medicament dispenser which: (1) effectively and efficiently dispenses and disperses dry powder medicament into the inhalation airstream of a user even when that inhalation airflow is at a rate that is less than the rate of an average person, (2) affords a highly efficient airflow within the dispenser to dispense and disperse the dry powder medicament, (3) wastes very little medicament, and (4) provides repeatable dosage accuracy.

According to one aspect of the present invention, there is provided a dry powder medicament dispenser for delivering a dose of micronized particles of a dry powder medicament to the respiratory system of a user. The dispenser comprises a housing, and means for providing a packed, predetermined, agglomerated dose of the dry powder medicament in a dosage chamber which is dimensioned to hold the predetermined dose of the dry powder medicament. The dispenser also comprises an internal pressure generator for generating a pressure for forcibly expelling the predetermined dose from the dosage chamber and external of the housing in micronized, deagglomerated form suitable for inhalation therapy. The dispenser has an effectiveness in deagglomerating the packed dose which is generally independent of user inhalation rate.

The device preferably comprises a dosage member having surfaces defining the dosage chamber. The dosage chamber defines a loading axis which is generally perpendicular to the surfaces defining an end of the dosage chamber. The means for providing a packed, predetermined agglomerated dose preferably comprises a medicament reservoir for storing a supply of dry powder medicament, and an agglomerator within the medicament reservoir for providing a positive, orientation independent packing force for loading the dry powder medicament from the medicament reservoir into the dosage chamber under pressure sufficient to pack the dry powder into a reproducible dose.

Most preferably the agglomerator is mounted for movement across the dosage chamber so that the positive, orientation independent packing force has a component that is generally parallel to the loading axis. The component of the packing force that is generally parallel to the loading axis progressively increases as the agglomerator moves across at least a portion of the dosage chamber.

Preferably, the pressure generator comprises a pressure chamber having a pressure outlet and a medicament delivery passageway. The pressure chamber suddenly provides a substantial fluid pressure differential across the packed dose so that the fluid flows to deagglomerate the packed dose into a plurality of respirable particles. More preferably the pressure generator provides a fluid flow at the dose from fluid which is initially generally free of velocity but which reaches a maximum velocity soon after the fluid begins to flow. At the maximum velocity, the fluid flow is turbulent and deagglomerates the packed dose into a plurality of respirable particles.

Also preferably, the pressure generator provides a fluid pressure differential to progressively increasing portions of the dose. Most preferably, the pressure differential is provided across the dose by releasing a pressurized fluid generally immediately adjacent the dose.

The dosage member, medicament reservoir, delivery passageway and pressure outlet are mounted for relative movement between (1) a load orientation with the dosage chamber opening into the medicament reservoir, and (2) a delivery orientation with the dosage chamber situated between the pressure outlet and the delivery passageway. Between the load and delivery orientations, progressively increasing portions of the dosage chamber, medicament delivery passageway and pressure outlet become aligned.

The present invention also embraces a use of the claimed dispenser for dispensing multiple individual doses of a dry powder medicament comprising the steps of (1) providing micronized particles of the medicament within a housing, (2) packing the micronized particles into a predetermined, agglomerated dose in a dosage chamber, and (3) generating a fluid pressure to forcibly expel the predetermined dose from the dosage chamber and external of the housing in micronized, deagglomerated form suitable for inhalation therapy.

There can be provided a cartridge which (1) is adapted to be received in and cooperate with a dry powder medicament dispenser comprising pressurization and actuation mechanisms adapted to minimize or reduce the inputs or operations required of a user; (2) may be replaced in the dry powder medicament dispenser with another cartridge after it is depleted; (3) affords re-use of the dispenser with a number of different cartridges; (4) may be constructed to restrict tampering with the dry powder medicament; and (5) optionally includes a counter assembly which affords an estimate of the number of dosages of medicament available in the cartridge.

The present invention will be further described with reference to the accompanying drawing wherein like reference numerals refer to like parts in the several views, and wherein:
Figure 1 is a perspective fragmentary view of one embodiment of dispenser according to the present invention shown from its front side;
Figure 2 is an exploded perspective view of the dispenser of Figure 1 rotated approximately ninety-degrees and showing a piston for pressurizing the fluid pressure chamber;
Figures 3 through 5 are enlarged sectional views of the device of the present invention taken approximately along lines 3-3 of Figure 1 with portions broken away to show details and which sequentially illustrate the delivery of medicament to a user;
Figures 5A through 5C are enlarged fragmentary perspective views of parts of the dispenser of Figure 1 which sequentially illustrate movement of a dosage chamber into alignment with a pressure outlet passageway;
Figure 5D is an enlarged fragmentary schematic illustration of initial alignment of a dosage chamber with the outlet passageway that occurs during the sequence illustrated in Figures 5A-5C;
Figure 6 is a perspective view of portions of a dosing member and a pressurization member which are preferably included in the dispenser of Figure 1;
Figure 7 is a perspective view of loading blades on a support shaft which are preferably included in the dispenser of Figure 1;
Figure 7A is a computer simulation illustrating sequential movement of a loading blade relative to a dosage member;
Figure 8 is a sectional view of a second embodiment of a dispenser according to the present invention;
Figure 9 is a sectional view of a third embodiment of a dispenser according to the present invention;
Figure 10 is an exploded perspective view of a cartridge;
Figure 11 is an enlarged perspective view of a partially assembled cartridge ;
Figure 12 is an enlarged perspective view of some of the elements of the cartridge shown in Figure 10 taken at a different angle than that of Figure 10 to show various details;
Figure 13 is a perspective view of the elements shown in Figure 12 taken at a different angle than that of Figure 12 to illustrate various details;
Figure 14 is an enlarged perspective view of elements of the cartridge shown in Figure 10 taken at a different angle to show details of portions of a counter assembly;
Figure 15 is an enlarged sectional perspective view of the cartridge shown in Figure 10 with the elements assembled;
Figure 16 is an enlarged sectional perspective view of the cartridge shown in Figure 10 similar to Figure 15 with parts omitted to show detail;
Figure 17 is another enlarged sectional perspective view of the cartridge shown in Figure 10 with the elements assembled and with a cover omitted to show detail;
Figure 18 is an enlarged sectional view of a cartridge assembled from the elements shown in Figure 10 with elements broken away and omitted to illustrate detail;
Figure 19 is an enlarged sectional view of a cartridge assembled from the elements shown in Figure 10 and including a sealing element between a metering sleeve and a pressure chamber; and
Figures 20 and 21 are schematic representations of an example of a medicament dispenser for use with the cartridge which sequentially illustrate the operation of the cartridge in conjunction with the dispenser.

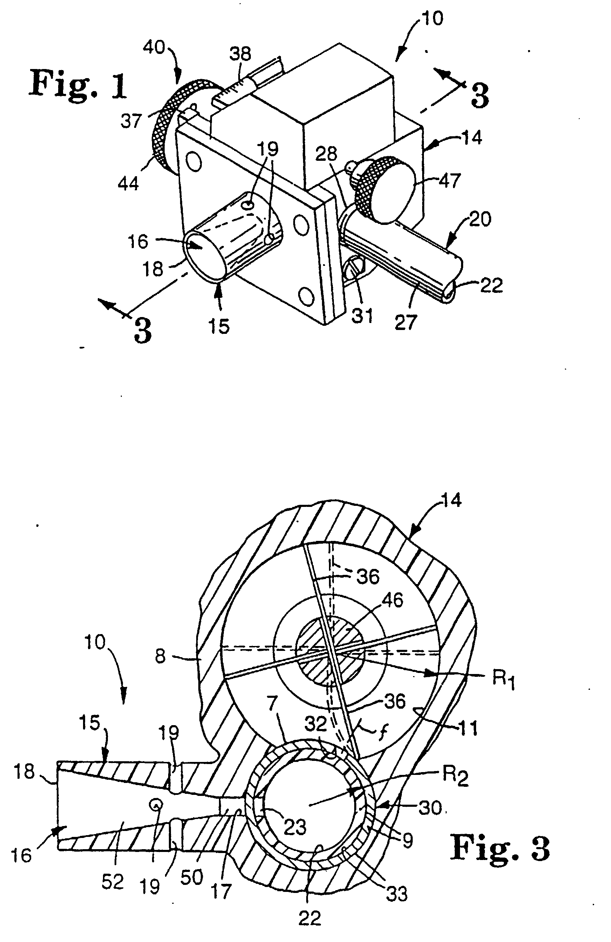

Referring now to Figures 1 through 7 of the drawing, there is shown one embodiment of dispenser according to the present invention, generally designated by the reference number 10. The dry powder medicament dispenser 10 delivers a dose of micronized particles of a dry powder medicament 12 to the respiratory system of a user.

The dispenser 10 has an effectiveness in deagglomerating the packed dose which is generally independent of .patient inhalation rate. Thus, a respirable mass of the dry powder medicament may consistently be provided to a patient, even at reduced patient inhalation airflow rates.

The dry powder medicament dispenser 10 comprises a housing 14 optionally including a mouthpiece portion 15. The dispenser 10 comprises means for providing a packed, predetermined, agglomerated dose of the dry powder medicament in a dosage chamber 32 which is dimensioned to hold the predetermined dose of the dry powder medicament.

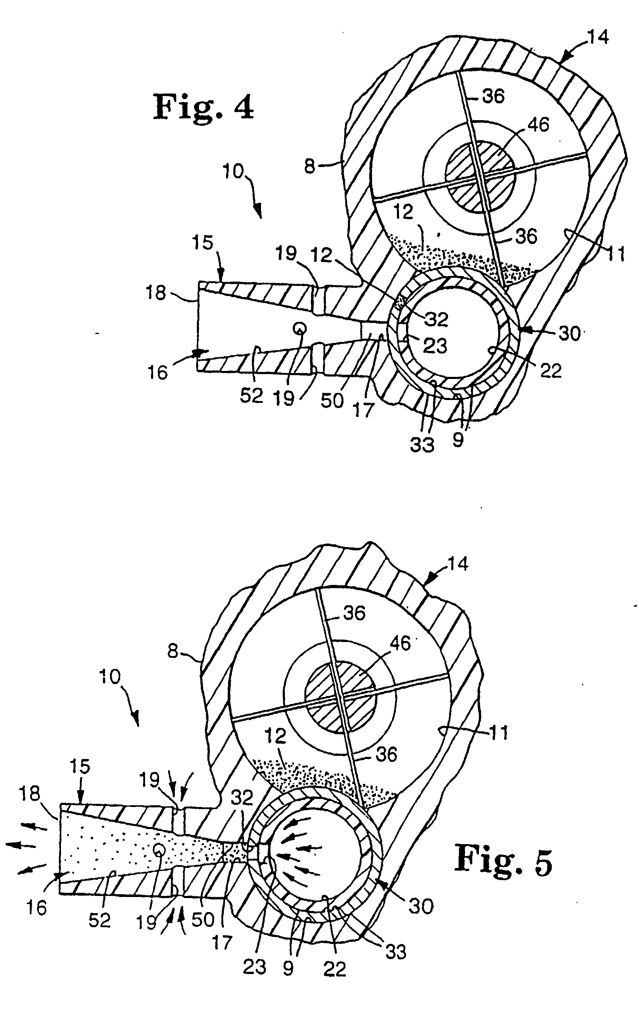

The device 10 preferably comprises a dosage member 30 having surfaces defining the dosage chamber 32. The dosage chamber 32 defines a loading axis f (Figure 3) which is generally perpendicular to the surfaces defining an end of the dosage chamber 32.

The means for providing a packed, predetermined agglomerated dose preferably comprises a medicament reservoir 11 for storing a supply of dry powder medicament 12, and an agglomerator within the medicament reservoir 11 for providing a positive, orientation independent packing force for loading the dry powder medicament 12 from the medicament reservoir 11 into the dosage chamber 32 under pressure sufficient to pack the dry powder 12 into a reproducible dose.

Most preferably the agglomerator is mounted for movement across the dosage chamber 32 so that the positive, orientation independent packing force has a component that is generally parallel to the loading axis f. The component of the packing force that is generally parallel to the loading axis f progressively increases as the agglomerator moves across at least a portion of the dosage chamber. The agglomerator is described in greater detail below.

The dispenser 10 also comprises an internal pressure generator 21 for generating a pressure for forcibly expelling the predetermined dose from the dosage chamber 32 and external of the housing 14 in micronized, deagglomerated form suitable for inhalation therapy. Preferably, the pressure generator 21 provides a fluid pressure differential to progressively increasing portions of the dose. This feature is described in detail below. The pressure differential is preferably provided by releasing a pressurized fluid generally immediately adjacent the dose. As used herein, the phrase, "immediately adjacent the dose" means that the dispenser 10 is substantially free of an obstruction or hindrance (e.g. an unpressurized (relative to the pressure provided by generator 21) region of ambient air or a tortuous or labyrinth like path) which may result in a pressure drop between the position of the release of the pressurized fluid and the dose or which may otherwise interfere with the communication of the pressure from the generator to the dose.

Also preferably, the pressure generator 21 generally instantaneously or suddenly provides a substantial fluid pressure differential across the packed dose so that the fluid flows to deagglomerate the packed dose into a plurality of respirable particles. As used herein, when it is said that the pressure generator suddenly provides a fluid pressure differential across the packed dose, it is meant that the dispener is free of any obstruction or hindrance that would substantially delay or slow the communication of the pressure from the pressure generator 21 to the dose.

The powdered medicaments 12 may be from any suitable therapeutic category such as, but not limited to antibiotics, proteins/peptides, steroids, bronchodilators, anticholinergics, lipoxygenase inhibitors, PAF antagonists, potassium channel activators, mast cell stabilizers, bradykinin analogs, enkephalins or interleukin. For example not intended to be limiting, the powder may be leuprolide, albuterol, insulin, pirbuterol, beclomethasone, terbutaline, salmeterol, fluticasone, tiamcinolone, salbutamol, isoproterenol, epinephrine, fenoterol, formoterol, procaterol, pentamidine, calcitonin, ipratropium, oxitropium, budesonide or their pharmaceutically acceptable salts.

The dry powder medicament dispenser 10 is particularly suited for delivery of a predetermined dosage unit comprising a plurality of very f ine particles having an average diameter as low as about 0.3 micrometers or even possibly less. Generally, by "very fine" or "micronized" particles, an average particle diameter is contemplated with a range of from about 0.3 micrometers to about 20 micrometers, and preferably from about 0.5 micrometers to about 6.0 micrometers. For example, a dose of the powdered medicament 12 may comprise 0.115 milligrams of a solid micronized albuterol sulfate powder having an average particle size of approximately 3 micrometers.

The housing 14 preferably has an outer surface 8 and inner surfaces. Those inner surfaces preferably comprise portions defining (1) a dosage member receiving chamber 9, (2) the medicament reservoir 11, (3) a medicament delivery passageway 16 extending between an injection inlet end 17 (e.g. a circular opening having a diameter of 1.57 millimeters 0.062 inches formed by a bore in the housing 14) communicating with the dosage member receiving chamber and an outlet end 18 opening through the mouthpiece portion 15, (4) at least one air entrance passageway 19 having an inlet end opening through the outer surface 8 of the housing 14 and an outlet end opening into the medicament delivery passageway 16, (5) a fluid pressure chamber 22, and (6) a pressure outlet passageway 23 with an inlet end communicating with the fluid pressure chamber 22 and an outlet end opening through the inner surface defining the dosage member receiving chamber generally opposite the injection inlet end 17 of the medicament delivery passageway 16. The housing 14 may be constructed from any suitable metal or polymeric material, such as but not limited to polyacetal, polyethylene, polypropylene, polycarbonates, or combinations thereof.

The medicament delivery passageway 16 preferably comprises a cylindrical turbulent flow portion 50 having a generally uniform cross section that is situated adjacent the injection inlet end 17 for affording a substantially turbulent flow of fluid from the fluid pressure chamber 22 to disperse the dry powder medicament 12 in the fluid. The medicament delivery passageway 16 optionally comprises a frusto-conical laminar flow portion 52 adjacent and diverging in cross sectional area toward the outlet end 18 of the medicament delivery passageway 16. The air entrance holes 19 open into the laminar flow portion 52 to afford laminar flow of fluid (preferably atmospheric air) and dry powder medicament in the laminar flow portion 52.

The inner surface portions defining the laminar flow portion 52 preferably diverge at an angle between approximately 15 and 30 degrees with respect to each other, preferably about 22 degrees. The embodiment of dispenser 10 shown in Figures 1 through 5 includes four circumferentially spaced cylindrical shaped air entrance holes 19 having axes generally transverse to or normal to the axis of the medicament delivery passageway 16.

The dispenser 10 includes the movable chamber means or dosage member 30 which may be a cylindrical tubular member having an inner diameter of 8.46 millimeters (0.333 inches) and a wall thickness of 0.46 millimeters (0.018 inches). Alternatively, the dosage member 30 may comprise a generally flat planar structure having a thickness of about 0.46 millimeters (0.018 inches).

In a preferred embodiment, the dosage member 30 comprises a dosage part having an outer sealing surface 33 and includes surfaces defining a dosage chamber 32 having the longitudinal axis f and extending through the dosage member 30 between spaced parts of the outer sealing surface 33 (see Figures 3-5). The dosage chamber 32 receives a dosage of dry powder medicament 12. The volume of the dosage chamber 32 is influenced by a variety of factors but is particularly influenced by the type of powdered medicament that is intended to be delivered. For example, when 0.115 mg. of albuterol sulfate is to be dispensed from the dosage chamber 32, the volume of the dosage chamber 32 should be approximately (1.45 X 10 (-5) cubic inches) 0.24 cubic millimeters . Also as an example, if the dosage chamber 32 is cylindrical, the dosage chamber may have a diameter of about 0.081 cm (0.032 inches) for a dosage member 30 that is 0.046 cm (0.018 inches) thick.

The cross-section of the dosage chamber 32 illustrated is circular to form a cylindrical passageway. Alternatively the dosage chamber 32 may have any suitable shape such as, but not limited to triangular, square, star, hexagonal, arcuate, polygonal, or any suitable shape form by combinations of straight and arcuate line segments. Preferably, there is a single dosage chamber 32 that provides a consistent dosage receiving volume which tends to receive consistent volumes of powdered medicament to thereby contribute to repeatable dosage accuracy.

The cross-section of the dosage chamber 32 may optionally be slightly smaller than the cross-section of the pressure outlet passageway 23 to afford complete expulsion of the medicament 12 from the dosage chamber 32, and to insure proper communication between the pressure outlet passageway 23 and the dosage chamber 32. For example, if the cross-section of the dosage chamber 32 is circular having a diameter of approximately (0.032 inches) 0.81 millimeters , then the cross-section of the pressure outlet passageway 23 may also be circular with a diameter of approximately (0.052 inches) 1.32 millimeters or (0.055 inches) 1.40 millimeters . Generally, the cross-section of the injection inlet 17 for the medicament delivery passageway 16 may have approximately the same or larger cross-sectional area than the cross-sectional area of the dosage chamber 32. For a circular cross-section of the dosage chamber 32 with a diameter of approximately 0.81 millimeters (0.032 inches), the cross-sectional area of the injection inlet 17 may also be circular with a diameter of approximately 1.57 millimeters (0.062 inches).

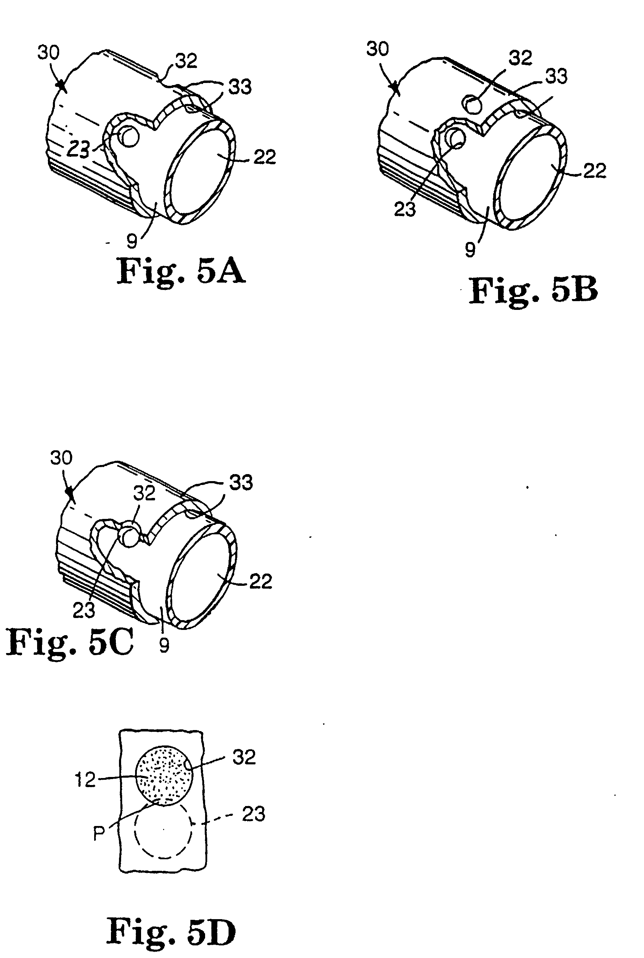

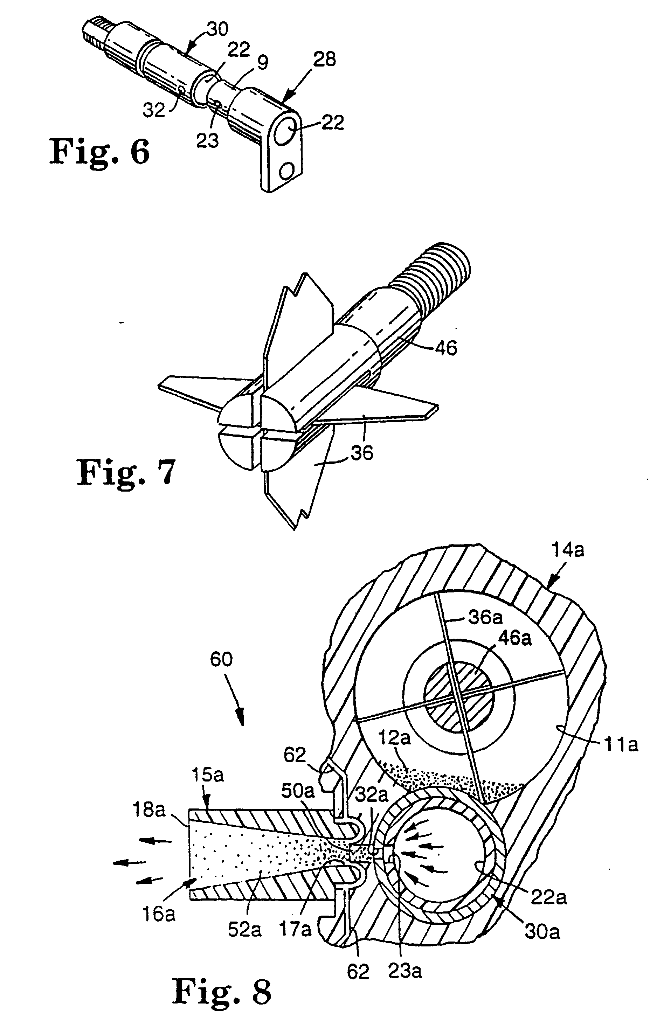

The dosage member 30 may be constructed from any suitable material such as, but not limited to, polymeric, plastic or metal materials or combinations thereof. The material used to construct the dosage member 30 and surfaces 9 should be sufficiently strong to resist deformation upon pressurization of a pressurization chamber 22. As best seen in Figures 5A-5C and 6, the dosage member 30 is preferably mounted adjacent portions of the inner surface 9 of the housing 14 which form the dosage member receiving chamber. Those portions of the inner surface 9 of the housing 14 which form the dosage member receiving chamber (see Figure 6) may comprise a part 28 which also forms portions of the fluid pressure chamber 22. Preferably the end of the part 28 adjacent gas pressure release aperture 23 has a closed end to form the pressurization chamber 22.

Means such as elastomeric sealing gaskets (not shown) may be provided to provide a seal between the dosage member 30 and the medicament reservoir 11 to prevent leakage or escape of powder 12 from the reservoir 11. Alternatively the means for preventing escape of powder 12 could comprise biasing means such as a screw for biasing the dosage member 30 into tight frictional engagement with the inner surface 9 of housing 14 adjacent outlet 7.

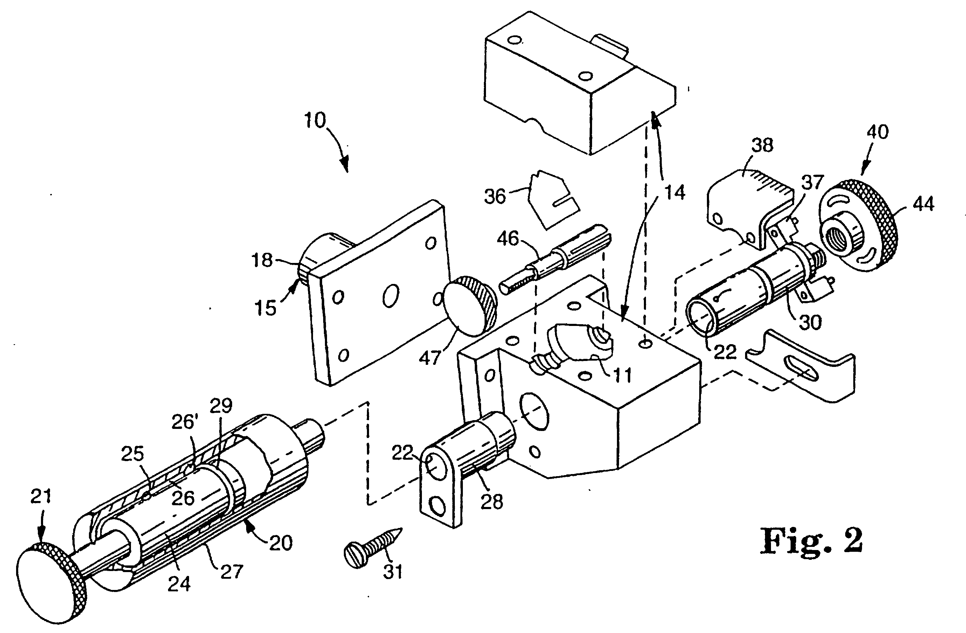

Preferably, the pressure generator 21 comprises a pressure chamber 22 having a pressure outlet 23 and the medicament delivery passageway 16. The pressure generator 21 (Figure 2) is provided for pressurizing fluid (e.g. gas such as ambient air) within the fluid pressure chamber 22 above ambient pressure and for retaining the fluid pressure within the fluid pressure chamber 22 above ambient pressure. The pressure outlet passageway 23 may be formed by a cylindrical bore in part 28. The part 28 may include a flange with an aperture to receive a screw 31 or any other suitable fastener to fix the part 28 in a proper position relative to the injection inlet opening 17 by firmly attaching the part 28 to the housing 14 so that the part 28 forms a portion of the inner surface 9 defining the dosage member receiving chamber. Alternatively the part 28 and the dispenser housing 14 may comprise a monolithic member such as an integrally molded member.

An example of a portion of the pressure generator 21 is illustrated in Figure 2 as a piston or plunger 24 having an O-ring 29 and a detent 25 adapted to be received in a channel or groove 26 in a f luid pressure member housing 27. An open end of the part 28 is adapted to receive a Luer fitting (not shown) or any suitable adapter for forming an air-tight attachment between the fluid pressurization member 20 and the part 28.

The groove 26 in the housing 27 has a first portion extending generally parallel to the axis of the fluid pressurization member housing 27 and a second locking portion 26' extending generally perpendicular to the first portion. To pressurize the fluid pressure chamber 22, a user first sealingly covers the pressure outlet passageway 23 with surfaces 33 of the dosage member 30 (Figures 3 and 4) and then moves the piston 24 axially within the gas pressurization member housing 27 toward the housing 14 until the detent engages the second locking portion of the groove 26'. The piston 24 may then be rotated counterclockwise (relative to the housing 14) to move the detent 25 into the second locking portion of the groove 26. The pressure within the fluid pressure chamber 22 biases the piston 24 away from the housing 27 and forces frictional engagement between the detent 25 and the locking portion of the groove 26' to retain the gas pressure within the gas pressure chamber 22 above ambient pressure.

While the means pressure generator 21 is shown in Figure 2 to include a piston 24, it should be noted that the pressure generator 21 may comprise any suitable means for pressurizing fluid (e.g. atmospheric air) within the fluid pressure chamber 22 above ambient pressure and for retaining the fluid pressure within the fluid pressure chamber 22 above ambient pressure including, but not limited to bellows, flexible bladders, or syringe/0-ring arrangements. Also, the means for locking the piston in a pressurization position may comprise any suitable means other than the described detent assembly.

The pressure generator 21 provides a fluid flow at the dose from fluid which is initially generally free of velocity but which reaches a maximum velocity soon after the fluid begins to flow. At the maximum velocity, the fluid flow in turbulent and deagglomerates the packed dose into a plurality of respirable particles. The pressure and volume within the pressurization chamber 22 provided by the generator 21 should be sufficient to completely expel all the powdered medicament 12 loaded into the dosage chamber 32.

While not intending to be bound by one theory, it is believed that the flow from a pressurized pressure chamber 22 to the medicament delivery passageway 16 may approximate flow through a nozzle or restriction such that there exists a critical pressure within the pressure chamber 22 which will provide a maximum velocity of flow within the medicament delivery passageway 16. Any additional pressure above the critical pressure will not result in fluid velocities in the medicament delivery passageway 16 above the maximum velocity.

Sufficient pressure and initial volume within the gas pressure chamber 22 is believed to assure the maximum velocity of fluid escaping from the pressure chamber 22 into the medicament delivery passageway 16. Generally, the higher the velocity of the fluid within the turbulence portion 50 the greater the turbulence in the turbulence portion 50. The greater the turbulence, the more the dispenser 10 disperses the powdered medicament 12 into smaller, more respirable particles. The turbulence results in high shear forces on the agglomerates of medicament 12 and causes collisions between the agglomerates of powdered medicament which tends to deagglomerate the large agglomerates into the desired primary particles. For example, the fluid pressure chamber 22 may be pressurized to a pressure of about 7.65 kg/cm² (108.8 pounds per square inch) absolute.

For a given size of dosage chamber 32, the pressure of fluid within the fluid pressure chamber 22 sufficient to expel the powdered medicament from the dosage chamber 32 is controlled by several factors, such as the size of the pressure outlet passageway 23, the initial volume of the gas chamber 22 before pressurization and the speed with which the dosage chamber 32 is moved across the pressure outlet passageway 23. Generally, for optimal dispensing, it is believed that a user should move the dosage chamber 32 across the pressure outlet passageway 23 as fast as possible. Optionally a biasing means such as a spring may be used to move the dosage chamber 32 across the pressure outlet 23.

Preferably, the entire cross sectional area of the dosage chamber 32 ultimately overlaps with the pressure outlet 23 and the total elapsed time between the time that (1) the dosage chamber 32 begins to open into the pressure outlet 23, until (2) the entire cross-sectional area of the dosage chamber 32 overlaps with the pressure outlet 23, is about four milliseconds.

When the pressure chamber 22 is pressurized, the medicament delivery passageway 16 remains at ambient or atmospheric pressure and there exists the potential to place a pressure differential across the packed dose when the dosage chamber 32 begins to overlap or open into the outlet 23. An example of the pressure generator 21 providing a substantial fluid pressure differential across the packed dose is that the pressure within the pressure chamber 22 should be at least about 1.1 times the ambient air pressure (the pressure in medicament delivery passageway 16). Preferably the pressure within the pressure chamber 22 should be at least about twice the ambient air pressure. The volume of pressurized fluid within chamber 22 should be enough to provide fluid flow during at least the time between when (1) the dosage chamber 32 begins to open into the pressure outlet 23, and (2) the entire cross-sectional area of the dosage chamber 32 overlaps the pressure outlet 23.

A manually-activatable means 40 (Figures 1 and 2) or optionally an automatic means mounts the dosage member 30 with at least portions of the outer sealing surface 33 in sealing engagement with the inner surface 9 defining the dosage member receiving chamber for movement from (1) a load position with the dosage chamber 32 generally aligned with the outlet opening 7 of the medicament reservoir 11, to (2) a delivery position with the dosage chamber 32 extending between the outlet end of the pressure outlet passageway 23 and the injection inlet end 17 of the medicament delivery passageway 16.

Preferably, the dosage member is in direct communication with the reservoir. By direct communication, it is understood that the dosage chamber 32 is directly exposed to the reservoir without intervention of any filter or any plurality of small orifices. In this way, it is possible to more optimally compact the entire amount of the drug into the chamber 32.

The means 40 may comprise an act ivation knob 44 (Figures 1 and 2) operatively connected to the dosage member 30 and having a flange 37 immovably affixed thereto. The housing 14 may have a stop flange 38 immovably affixed thereto. Rotation of the knob 44 causes the flange 37 to move relative to the stop flange 38. The stop flange 38 has surfaces adapted to abut the knob flange 37 when the dosage member 30 is moved between the load and delivery positions. Abutment between the one side of the stop flange 38 and the flange 37 ensures that the dosage chamber 32 moves to the correct position for dispensing relative to the pressure outlet passageway 23 and injection inlet 17, and abutment between the other side of the stop flange 38 and the flange 37 ensures that the dosage chamber 32 is in a position to receive powdered medicament 12 from the reservoir 11.

At the load position (Figure 3) the inner surface 9 defining the dosage member receiving chamber seals the end of the dosage chamber 32 opposite the medicament reservoir opening 7, and the outer sealing surface 33 of the dosage member part 32 seals shut the outlet end of the pressure outlet passageway 23. Portions 33 of the dosage member 30 seal shut the outlet end of the pressure outlet passageway 23 during an initial part of the movement of the dosage member 30 from the load position (e.g. Figure 3) to the delivery position (e.g. Figure 5). During a final part of the movement from the load position to the delivery position, progressively increasing portions of the pressure outlet passageway 23, the dosage chamber 32, and the injection inlet 17 move into alignment to afford dispersion of the dry powder medicament 12.

When (1) the dosage member 30 is positioned at the load position so that medicament 12 from the medicament reservoir 11 may be moved into the dosage chamber 32, (2) the generator means 21 is activated to pressurize fluid within the pressure chamber 22, and (3) the dosage member 30 is then moved to the delivery position while a user is inhaling air through the air entrance passageway 19, pressurized fluid will pass from the pressure chamber 22 through the pressure outlet passageway 23 and discharge the powdered medicament 12 from the dosage chamber 32 into the air stream being inhaled by the user through the air entrance passageway 19.

While the dosage chamber 32 has been described as moving relative to the housing 14 and the reservoir 11, it should be noted that any of the dosage member 32, medicament reservoir 11, delivery passageway 16 or the pressure outlet 23 may be mounted for movement so long as (1) in the load position or orientation, the dosage chamber 32 opens into the medicament reservoir 11, and (2) in the delivery position or orientation, the dosage chamber 32 is situated between the pressure outlet 23 and the delivery passageway 16. Optionally, the delivery passageway 16 may be omitted.

Figures 5A, 5B and 5C sequentially illustrate portions of the pressure outlet passageway 23 and the dosage chamber 32 moving into alignment. Figure 5D illustrates the dosage chamber 32 as it initially opens into the pressure outlet passageway 23.

When the dosage chamber 32 initially opens into the pressure outlet 23, a pressure differential is provided across the dose. There is generally very little or no pressure loss between the position of release of the pressurized fluid within chamber 22 and the dose. The pressure differential provided across the dose by the pressure within the pressure chamber 22 and ambient pressure in the delivery passageway 16 is preferably the maximum pressure differential that may be provided across the dose for those pressures. Thus, pressure within the pressure chamber 22 is efficiently used by the dispenser 10. Alternatively, the pressure provided by the generator which forcibly expels the dose from the dosage chamber 32 may be provided by a vacuum across the dose.

The feature of the present invention wherein the pressure outlet passageway 23 is situated directly or immediately adjacent the dosage chamber 32 during dispensing of the medicament 12 is believed to contribute to complete dispersion of the medicament 12 within the dosage chamber 32. This "eclipse" motion of the dosage chamber 32 relative to the pressure outlet passageway 23 while pressure chamber 22 is pressurized is believed to effectively remove and deagglomerate the powder in dosage chamber 32. The eclipse motion of the dosage chamber relative to the pressure outlet is believed to afford complete, efficient dispersion of the dry powder medicament, and provides a cloud of respirable sized powdered medicament that is easily inhaled by a user, even at an inhalation airflow that is less than the rate at which an average person may inhale.

The portion P (See Figure 5D) of the dosage chamber 32 that initially opens into the pressure outlet passageway 23 and the injection inlet 17 is believed to be "blasted" from the dosage chamber 32 with the remaining powder following shortly thereafter. The pressure immediately at the location of the powder 12 is believed to effectively disperse and dispense the powder 12 from the dosage chamber 32 to the delivery passageway 16.

Dispensing a powdered medicament 12 in the manner according to the present invention reduces the amount of pressure required to completely dispense the powder 12. The pressurization chamber 22 need only be pressurized with enough pressure to (1) deagglomerate the powder 12, and (2) expel the medicament 12 from the dosage chamber 32 and into the medicament delivery passageway 16. The pressure within the pressurization chamber 22 is not required to transmit all of the powder into the mouth of the user. The user's inhalation through air entrance hole 19 subsequently draws the dispersed powder completely through the medicament delivery passageway 16 and into the lungs of the user.

### POWDER LOADING ASSEMBLY

The dry powder 12 tends to adhere to the walls of the medicament reservoir 11 and to form unduly large agglomerates. The dispenser 10 according to the present invention utilizes a novel powder loading assembly that contributes to consistent dosage accuracy. The powder loading assembly includes means for providing a packed, predetermined, agglomerated dose of the dry powder medicament in a dosage chamber 32 which is dimensioned to hold the predetermined dose of the dry powder medicament. The means cause transfer of the dry powder medicament 12 from the medicament reservoir 11 to the dosage chamber 32 in the dosage member 30 when the dosage member 30 is in the load position.

The means for providing a packed, predetermined agglomerated dose preferably comprises the medicament reservoir 11, and an agglomerator within the medicament reservoir 11 for providing a positive, attitude or orientation independent packing force for loading the dry powder medicament 12 from the medicament reservoir 11 into the dosage chamber 32 under pressure sufficient to pack the dry powder 12 into a reproducible dose.

When the dispenser 10 is used to dispense micronized particles of a dry powder drug 12, the micronized particles tend to resist flow from the medicament reservoir 11 into the dosage chamber 32. The packing force is described as positive because it is independent of or greater than ambient forces such as van der Waals forces between the micronized particles, or the effect of gravity on the micronized particles. The packing force is said to be orientation independent because the powder loading assembly will reproducibly load the medicament 12 into the dosage chamber 32 even in an upside down or inverted orientation.

The effect of the packing means of the present invention is to pack the powdered medicament into the dosage chamber. Packing the dosage chamber is believed to contribute to repeated, consistent dosage accuracy as the chamber 32 is consistently packed with the same amount of powder.

Preferably, the structure comprises flexible loading blades 36 each having proximal and distal 39 ends and a leading surface between the proximal and distal ends. The powder loading assembly includes at least one and preferably four flexible powder loading blades 36 mounted on a shaft or core 46 having generally the same axis as the axis of the medicament reservoir 11. Means 40 (Figure 2) drives the blade 36 across the surfaces of the medicament reservoir 11.

The blade turning shaft 46 is rotated by a powder loading knob 47. The blades 36 are constructed from any suitable flexible material including but not limited to polymers, metals or polyesters. Rotation of the knob 47 while the dosage chamber 32 opens into the medicament reservoir causes revolving movement of the blades 36 that scrapes medicament 12 from the walls of the reservoir 11, simultaneously loads the dosage chamber 32 with a dosage of dry powder medicament 12 and also tends to agitate the powder 12 to break the powder 12 into smaller agglomerates which are more readily loaded into the dosage chamber 32.

The shaft or core 46 moves the flexible blades 36 along a predetermined path within the medicament reservoir 11 with the leading surface leading and the distal end 39 of the blade 36 moving along a portion of the inner surface defining the medicament reservoir 11 during a first portion of the predetermined path (Figure 3, solid line). During a second portion (Figure 3 dashed lines) of the predetermined path, the distal end 39 of the blade 36 moves along a portion of the outer sealing surface 33 of the dosage member 30 which results in progressively increasing bending of the blade 36 to form the leading surface into a convex surface and to move the leading surface progressively closer to the outer sealing surface 33 of the dosage member 30.

When the distal end 39 of the blade 36 is described as moving along a portion of the outer sealing surface of the dosage member, it is herein contemplated that a thin layer of powder 12 e.g. 0.025 cm (0.01 inches) may be present between the distal end of the blade 36 and the dosage member 30.

The medicament reservoir 11 is generally cylindrically concave and has a medicament reservoir axis which defines a reservoir radius R1. The outer surfaces of the dosage member 30 along with its axis define a radius R2. The core 46 mounts the proximal ends of the blades 36 for revolving movement around the medicament reservoir axis. The portion of the outer sealing surface 33 of the dosage member 30 along which the distal end of the blade 36 moves is cylindrically concave about an axis generally parallel to the medicament reservoir axis R1.

The distance between the axes of the blade 36/medicament reservoir 11 and the dosage member 30 is less than the sum of the radii of the dosage member 30 and the medicament reservoir 11 (R1 + R2) to afford interference between the powder loading blade 36 and the dosage member 30 during the second portion of the predetermined path so that the powdered medicament 12 is loaded into dosage chamber 32 in a direction that is generally in the direction of the longitudinal or loading axis of the dosage chamber 32.

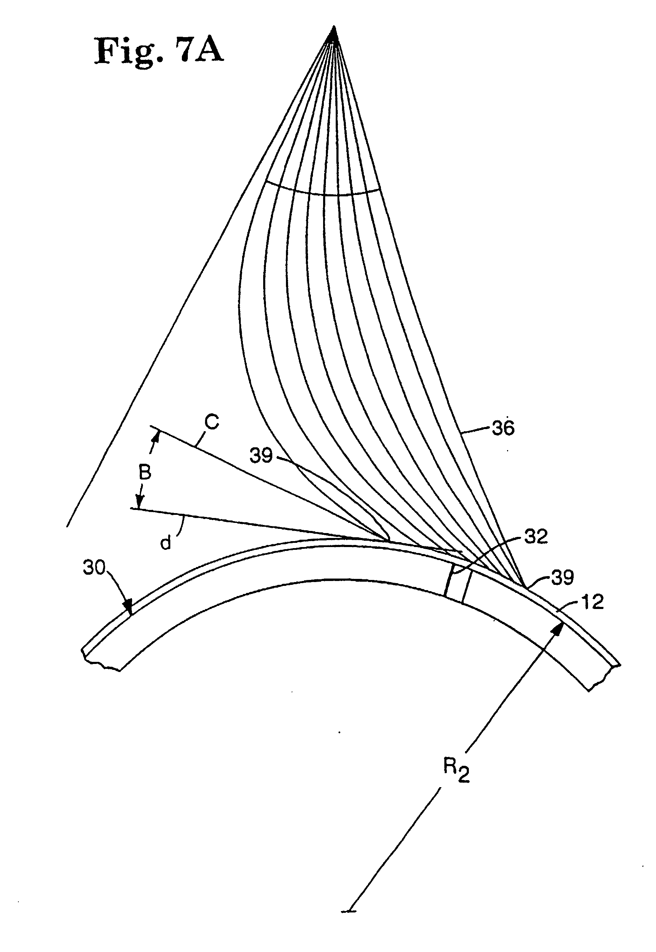

Figure 7A depicts a computer simulation of the powder loading assembly according to the present invention. To generate the computer simulation, a powder layer 12 of 0.025 cm (0.01 inches) was assumed to be present between the blade 36 and the dosage member 30. The simulation illustrates the position of the blade 36 for equal increments of rotation of core 46. When the flexible blade 36 engages the dosage member 30, the blade 36 will bend. At the line of contact between the blade 36 and the sealing surface of the dosage member 30 (or a thin layer of powder 12 directly adjacent thereto), there is an imaginary blade tangent line c that is tangent to the leading surface of the blade 36 and an imaginary dosage member tangent line d tangent to the outer sealing surface of the dosage member 30. The blade tangent line c and the dosage member tangent line d form a blade tangent angle Beta therebetween which progressively decreases as the blade 36 moves along the sealing surface and across the dosage chamber 32.

The loading assembly according to the present invention tends to beneficially pack the powdered medicament 12 into the dosage chamber 32. Just after the blade 36 encounters the dosage member 30, the action is similar to a rolling motion. As the blade moves across the dosage chamber 32, the blade tangent angle Beta progressively decreases so that the powdered medicament 12 is loaded into dosage chamber 32 in a direction that is generally along the loading axis f of the dosage chamber 32 (e.g. shown in Figure 3 by the dashed lines, and Figure 7). Forcing the powdered medicament in a direction generally along the axis f of the dosage chamber 32 is believed to repeatedly load the chamber with substantially uniform amounts of medicament 12 to thereby contribute to dosage accuracy. As an example which is not intended to be limiting, the dosage member 30 may have a radius R2 of approximately 0.475 cm (0.187 inches), the medicament reservoir 11 may have a radius R1 of approximately 0.81 cm (0.32 inches), and the distance between the axes of the medicament reservoir 11 and the dosage member 30 may be about 1.19 cm (0.47 inches). The powder loading blades 36 may have a length from the axis of the medicament reservoir 11 of approximately 0.8 cm (0.315 inches) and are constructed from a flexible material such as polyester to ensure that the blade will deflect to the position shown in Figure 3 by the dashed lines.

Preferably, at least one of the loading blades 36 has a raking surface comprising a V-shaped notch (Figure 7) in its distal end 39 that disperses agglomerates of the dry powder medicament 12 into smaller particles and separates the dry powder 12 from the walls of the medicament reservoir 11.

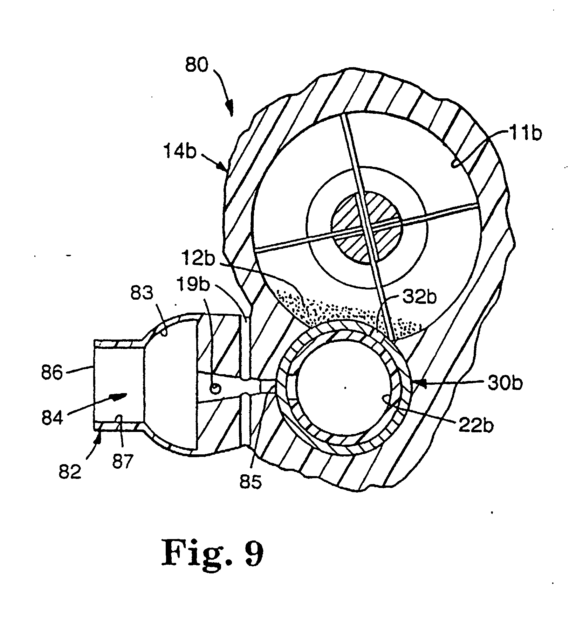

Figure 8 illustrates a second alternative embodiment of a dispenser according to the present invention designated by the reference character 60 which has many parts that are essentially the same as the parts of the dispenser 10 and which have been identified by the same reference number to which the suffix "A" has been added.

Like the dispenser 10 described in Figures 1 through 5, the dispenser 60 shown in Figure 8 comprises a housing 14A defining a medicament reservoir 11A which is adapted for storing micronized dry powder medicament 12A, and a mouthpiece 15A having surfaces defining a medicament delivery passageway 16A having an injection inlet opening 17A and an outlet opening 18A for passage of a respirable dose of the dry powder 12A for subsequent delivery to a user. A gas pressure chamber 22A adapted to be pressurized above ambient pressure and a movable dosage member 30A having surfaces defining a dosage chamber 32A are also provided.

Unlike the dispenser 10, the dispenser 60 includes arcuate air entrance holes 62 which are positioned to afford airflow into the medicament delivery passageway 16A at an angle relative to the axis of the passageway which is much less than ninety-degrees, preferably approximately zero (0) degrees. The position configuration of the air entrance holes 62 shown in Figure 8 is believed to provide a more laminar flow of air to a user.

Figure 9 illustrates a third alternative embodiment of dispenser according to the present invention designated by the reference character 80 which has many parts that are essentially the same as the parts of the dispenser 10 and which have been identified by the same reference number to which the suffix "B" has been added.

Like the dispenser 10 described in Figures 1 through 5, dispenser 80 shown in Figure 9 comprises a housing 14B defining a medicament reservoir 11B for storing micronized dry powder medicament 12B, air entrance holes 19B, a mouthpiece portion 82, a gas pressure chamber 228 adapted to be pressurized above ambient pressure and a movable dosage member 30B having surfaces defining a dosage chamber 32B are also provided.

Unlike the dispenser 10, the inner surfaces of the dispenser 80 include a medicament delivery passageway 84 having an injection inlet opening 85 and an outlet opening 86 for passage of a respirable dose of the dry powder 12B for subsequent delivery to a user. The medicament delivery passageway 84 has a deceleration portion 83 which has a larger cross sectional area than its turbulent and laminar flow portions and which is preferably semispherically shaped. The deceleration portion affords rapid expansion and loss of kinetic energy of the powder 12 after it exits the dosage chamber 32b. The deceleration portion 83 restricts passage of unduly large agglomerates of medicament 12 to deter such agglomerates from becoming entrained in the back of the user's throat. The semispherical chamber 83 may have a radius between about 0.51 cm and 1.78 cm (0.2 and 0.7 inches) and may be connected to a cylindrical passageway 87 which may have a radius between about 0.51 cm and 1.27 cm (0.2 and 0.5 inches).

### OPERATION

The present invention may also be described as a method of dispensing multiple individual doses of a dry powder medicament 12 comprising the steps of (1) providing micronized particles of the medicament 12 within a housing 14, (2) packing the micronized particles 12 into a predetermined, agglomerated dose in a dosage chamber 32, and (3) generating a fluid pressure to forcibly expel the predetermined dose from the dosage chamber 32 and external of the housing 14 in micronized, deagglomerated form suitable for inhalation therapy.

More particularly, the operation of the present invention will now be explained using the dispenser 10 as an example. First, a user should position the dosage chamber 32 in a position to receive medicament 12 from the reservoir 11. The user may rotate knob 44 until flange 37 abuts one side of atop flange 38. This position is designed to place the dosage chamber 32 in the position shown in Figure 3. At this position, the user may rotate knob 47 to rotate blades 36 to thereby load powdered medicament 12 into the dosage chamber 32.

Either before or after loading the dosage chamber 32 with medicament 12, the gas pressure chamber 22 should be pressurized by pushing the piston 24 toward the housing 14 and rotating the piston 24 to move the detent 25 into the groove 26' to retain the pressure in the chamber 22. During pressurization of chamber 22, the dosage member 30 may be in any position, such as either the position illustrated in Figure 3 or the position illustrated in Figure 4, as long as the dosage chamber 32 does not open into the pressure outlet passageway 23 and injection inlet 17.

Once the dosage chamber 32 is loaded with powdered medicament 12 and the pressurization chamber 22 is pressurized, the user may then actuate the dispenser 10 by rotating the knob 44 as quickly as possible until flange 37 abuts the other side of stop flange 38. This position is designed to place the dosage chamber 32 in the position shown in Figure 5. As the powder filled dosage chamber 32 initially begins to open into the pressure outlet passageway 23, the pressure within the gas pressure chamber 22 is highly concentrated over the portion of the dosage chamber 32 that is initially opening into the pressure outlet passageway 23 (Figure 5D). Such concentration of pressure at the location of the powder 12 is believed to effectively disperse and dispense the powder 12 from the dosage chamber 32 to the delivery passageway 16.

Either immediately before, during or just after moving the dosage chamber 32 to the position shown in Figure 5, the user should inhale creating an air flow through air entrance passageway 19 and through portions of medicament delivery passageway 16. The inhalation is preferably generally synchronous with rotation of knob 44 to ensure delivery of the powder 12 to the user as is appropriate with the pulmonary target. However, it is believed that some time may pass before inhalation without an unduly large loss of dispenser efficiency. The pressure within the gas pressure chamber 22 is generally not sufficient to transport the powder 12 completely into the mouth or nose of a user as is appropriate with the pulmonary target. However, once the powder 12 is expelled from dosage chamber 32, a user should ultimately inhale to effectively transmit the powder 12 to the user's lungs.

### CARTRIDGE ASSEMBLY

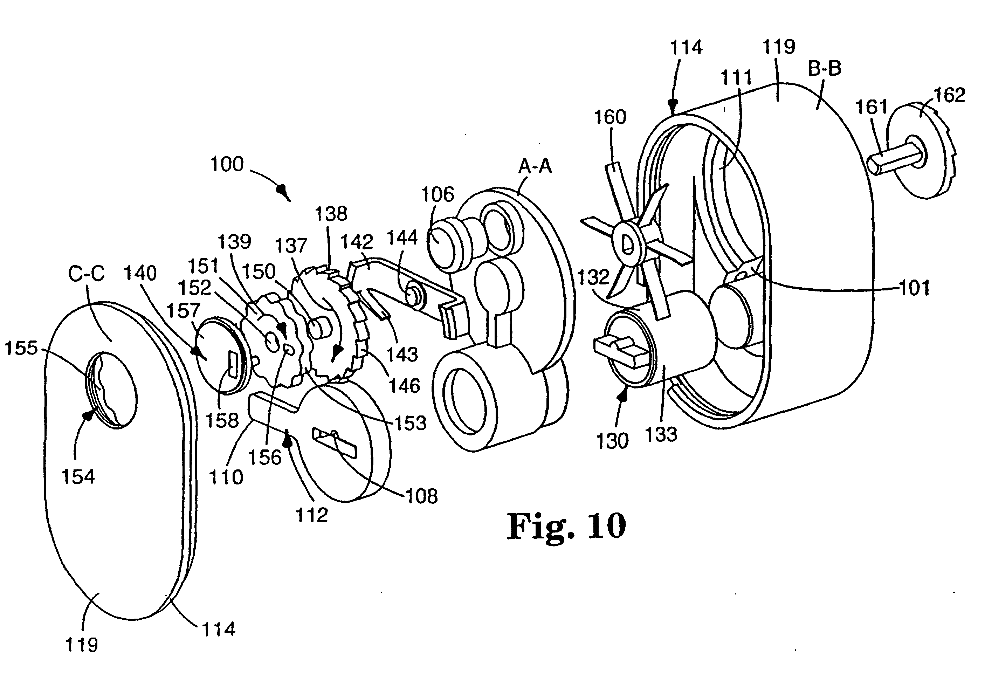

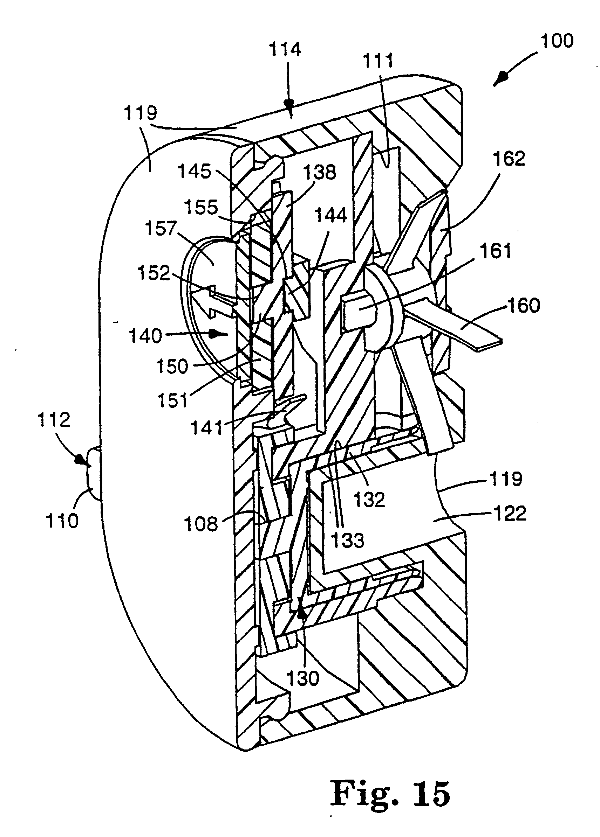

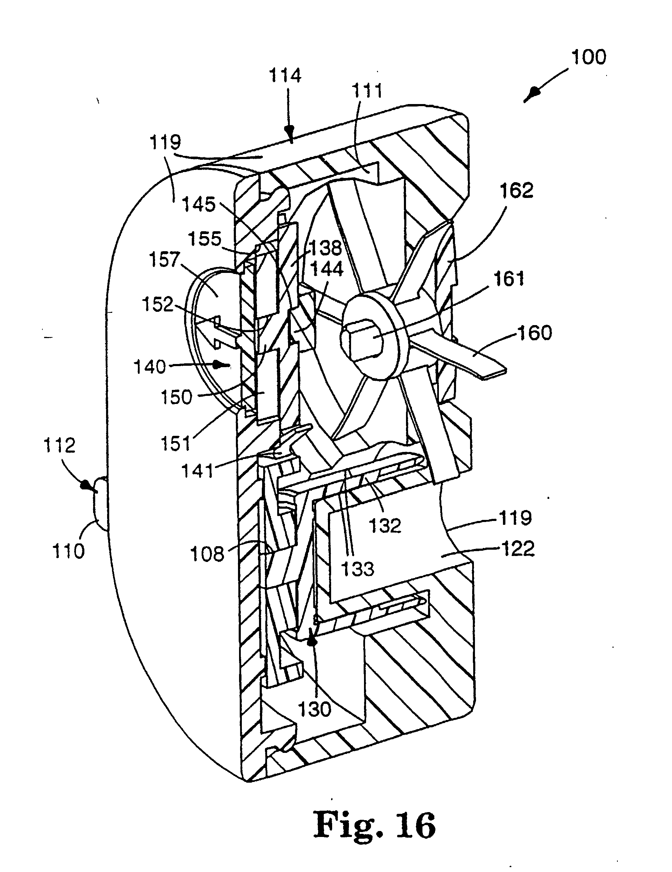

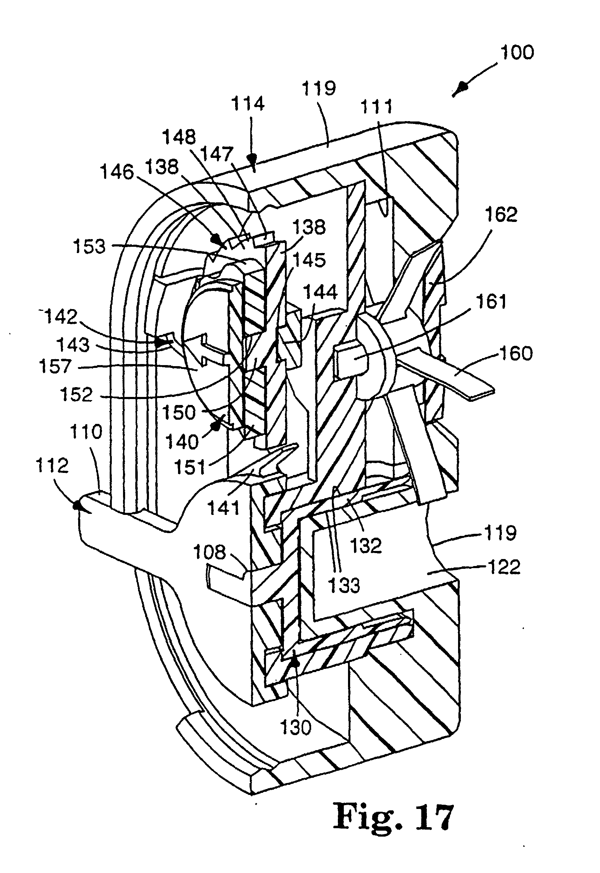

Referring now to Figures 10 through 21 of the drawing, there is shown a preferred embodiment of cartridge generally designated by the reference character 100.

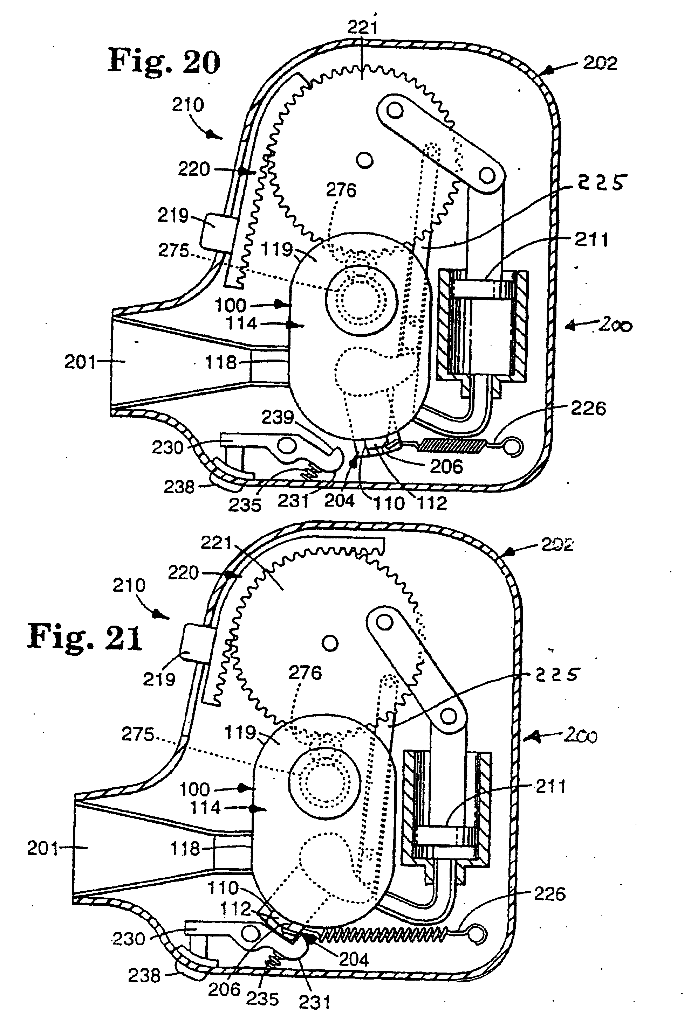

The cartridge 100 is received in a dry powder medicament dispenser 200 (Figures 20 and 21) having a mouthpiece portion 201, and actuation means 210 including pressurization means 211 to be explained in greater detail later.

The cartridge 100 may be constructed to be relatively inexpensive and disposable. Thus, when the medicament within a cartridge 100 is depleted, the cartridge 100 is replaced with a different cartridge and the depleted cartridge is disposed of. Such an arrangement affords re-use of the relatively expensive dispenser 200 with a number of different cartridges 100.

The cartridge 100 comprises a cartridge housing 114 including outer surfaces 119 adapted to be received in the dry powder dispenser 200. The housing 114 may be constructed from a material similar to the material used to construct the housing 14 and is preferably constructed from any suitable material approved by the U.S. Food and Drug Administration for medical purposes, such as the polycarbonate grade # HP-1 LEXAN T.M., generally available from General Electric of Pittsfield Massachusetts.

Figure 10 illustrates three separate major elements that are included in the assembly forming the cartridge 100. The major elements include a cartridge base BB, an intermediate portion AA and a cover CC. Preferably the parts AA, BB and CC may be press and/or snap fit together to form parts of the cartridge 100. Additionally, the cover CC may be adhesively adhered to the base BB to restrict tampering and access to medicament within cartridge 100. It should be noted that while the housing 114 is described as preferably comprising three major parts AA, BB and CC, alternatively the housing 114 may be comprised of fewer or additional major parts.

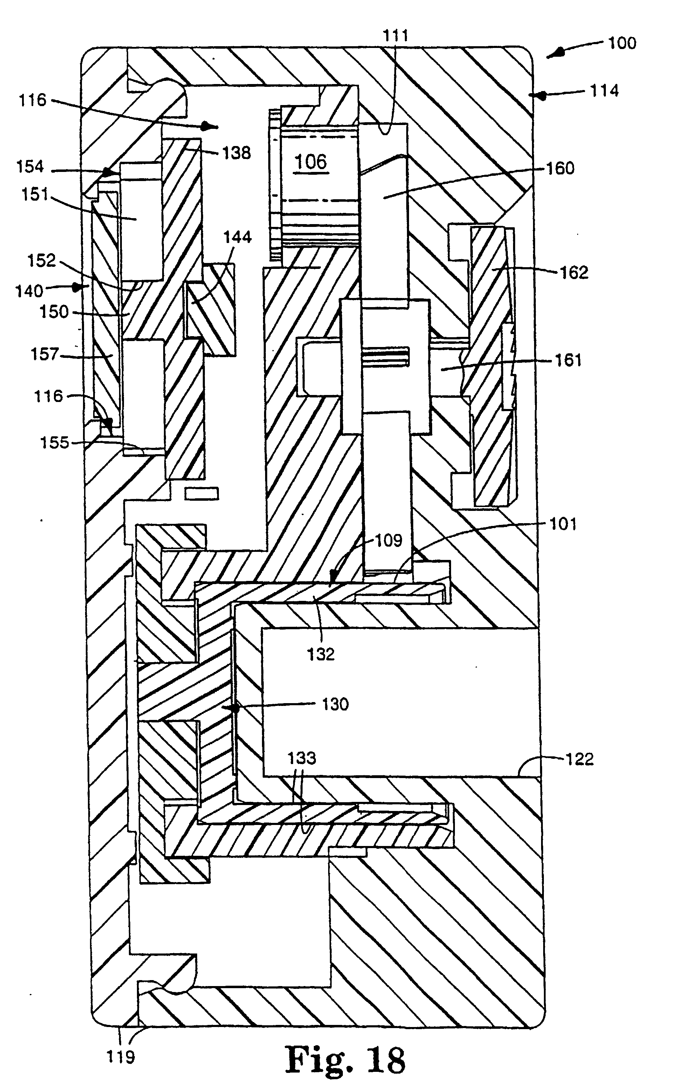

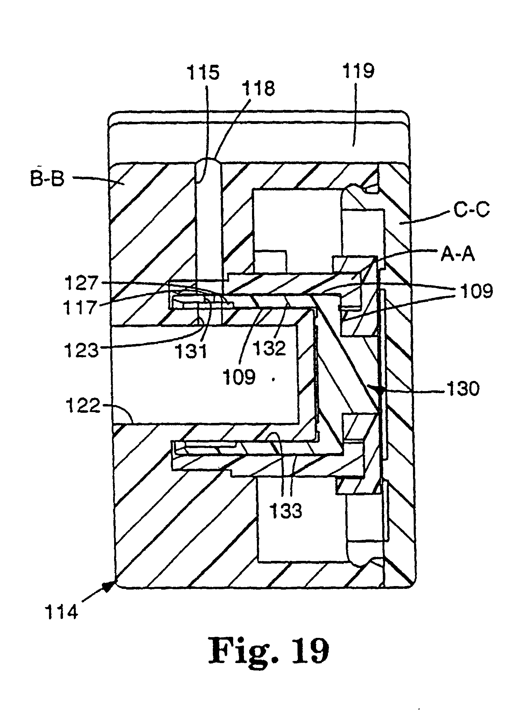

The housing 114 includes inner surfaces comprising a dosage member receiving chamber 109 (Figure 19), a counter assembly receiving cavity 116 (Figure 18), and a medicament reservoir 111 for storing dry powder medicament (e.g. the medicament 12 described above in the description of the dispenser 10). The medicament reservoir 111 has a loading aperture 101 (Figure 18) communicating with the dosage member receiving chamber 109. As an example not intended to be limiting, the medicament reservoir 111 may be semi-cylindrical shaped as shown in Figures 10, and 15-18 and may include an outer diameter of about 19.3 millimeters and a width of about 2.03 millimeters. A desiccant plug 106 may be attached to intermediate portion AA to cover an access aperture to reservoir 111. The desiccant plug 106 removes moisture from the medicament 12. It should be noted that powder may also be initially loaded into the reservoir 111 through aperture 101.

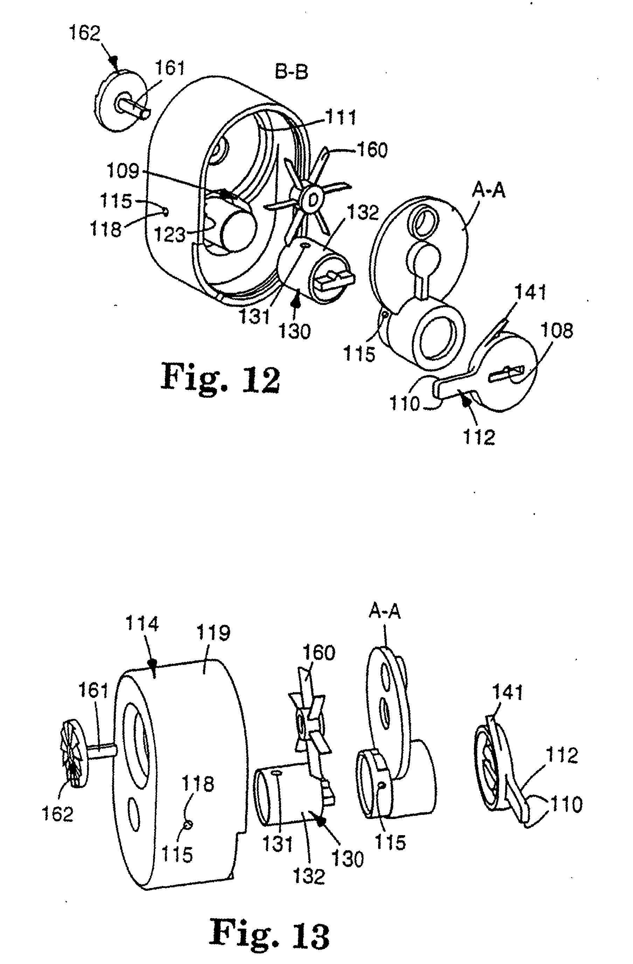

The inner surfaces of the housing 114 also include a medicament release bore 115 (e.g. a cylindrical bore having a diameter of about 1.6 millimeters and a length of about 3.2 millimeters) extending between an outlet end 118 at the cartridge housing 114 outer surfaces 119 and an injection inlet end 117 communicating with the dosage member receiving chamber 109. As shown in Figures 20 and 21, the outlet end 118 communicates with the mouthpiece portion 201 of the medicament dispenser 200. Also, as shown in Figures 12, 13 and 19, the medicament release bore 115 extends through portions of both major parts AA and BB.

Additionally, the inner surfaces of the housing 114 include a pressure chamber 122 (e.g. a cylindrical chamber having a diameter of the inner surfaces of the chamber of approximately 5.82 millimeters) that opens to the outer surfaces 119 of the housing 114. The pressure chamber 122 is operatively connected to the pressurization means 211 of the medicament dispenser 200 by, for example, a suitable sealing means (e.g. an elastomeric or rubber ring or washer, not shown) press fit between the housing 114 and the dispenser 200.

The pressure chamber 122 is pressurized by the pressurization means 211 of the medicament dispenser 200. For example, the pressurization means 211 may comprise a piston/cylinder arrangement as shown in Figures 20 and 21. Alternatively, the pressurization means 211 may comprise any suitable pressurization means for pressurizing fluid such as but not limited to bellows or flexible bladders.

Figure 19 illustrates that the inner surfaces of the housing 114 include a pressure release bore 123 (e.g. a cylindrical bore having a diameter of about 1.6 millimeters and having a length of about 1.01 millimeters) having a first end communicating with the pressure chamber 122 and a second end opening through the inner surface defining the dosage member receiving chamber 109 generally opposite the injection inlet end 117 of the medicament release bore 115.

The cartridge 100 includes a dosage member 130 mounted within the dosage member receiving chamber 109. The dosage member 130 includes a cylindrical, tubular dosage part 132 having sealing surfaces 133 and having a dosage chamber 131 extending through the dosage part 132 between spaced parts of the sealing surfaces 133. The dosage member 130 may be constructed of a material similar to the material used to construct the dosage member 30 and is preferably a material approved by the U.S. Food and Drug Administration for medical purposes such as, but not limited to, Grade #M90 SELCON T.M. generally available from HOECHST CELANESE.

The dosage chamber 131 receives a dosage of dry powder medicament from the medicament reservoir 111. The volume of the dosage chamber 131 is influenced by a variety of factors similar to the factors affecting the volume of the dosage chamber 32, particularly the type of medicament that is intended to be delivered. As an example not intended to be limiting, the dosage member 130 may comprise a sleeve with dimensions similar to those given in the example of the dosage member 30 described above.

The cross-section of the dosage chamber 131 may vary similar to the cross-section of the dosage chamber 32 mentioned above. Preferably, there is a single dosage chamber 131 to provide a consistent dosage volume which tends to receive consistent amounts of powdered medicaments to thereby contribute to dosage accuracy.

Like the dispenser 10, in the cartridge 100, means such as elastomeric or rubber sealing gaskets (not shown) may optionally be disposed to provide a seal between the dosage member 130 and the medicament reservoir 111, generally at the aperture 101.

Additionally, Figure 19 illustrates a sealing means added to the elements of the cartridge 100 shown in Figure 10. The sealing means comprises, for example, a biasing rubber or elastomeric member 127 attached between the dosage member 130 and the pressure release bore 123/pressure chamber 122 to provide a seal between the pressure release bore 123/pressure chamber 122 and the dosage member 130. The sealing means 127 may be simultaneously injection molded with the remaining portions of the pressure chamber 122 portion of the housing or may be adhered thereto with an appropriate adhesive. As shown in Figure 19, the sealing means 127 includes a bore extending therethrough so that the dosage chamber 131 and medicament release bore 115 communicate with the pressure release bore 123.

The cartridge 100 also includes an actuation arm 112 connected to the dosage part 132 and having portions extending beyond the outer surfaces 119 of the cartridge housing 114. The actuation arm 112 includes surfaces 110 adapted to be manipulated by the actuation means 210 of the medicament dispenser 200. The actuation arm 112 may be connected to the dosage member in any suitable manner such as by a snap fit with a detent groove 108 to afford proper orientation of the arm 112 relative to the dosage member 130.

The actuation arm 112 moves the dosage member 130 from (1) a load position (e.g. Figure 21) with the dosage chamber 131 opening into the loading aperture of the medicament reservoir 111, to (2) a delivery position (Figure 20). The movement between the load and delivery positions of the dosage member 130 relative to the reservoir 111, injection inlet 117 and the pressure release bore 123, is similar to the movement between the load and delivery positions of the dosage member 30 relative to the reservoir 11, gas pressure release aperture 23 and the injection inlet 17 of the dispenser 10 shown in Figures 3, 5, and 5A through 5D as discussed above. Like the dispenser 10, in the cartridge 100, at the load position, a portion of the inner surfaces defining the dosage member receiving chamber 109 seals the end of the dosage chamber 131 opposite the medicament reservoir 111, and a portion of the sealing surfaces 133 of the dosage member 130 seals shut the second end of the pressure release bore 123. At the delivery position, the dosage chamber 131 extends between the second end of the pressure release bore 123 and the injection inlet end 117 of the medicament release bore 115.

Portions of the inner surface defining the dosage member receiving chamber 109 seal shut both ends of the dosage chamber 131 and portions of the sealing surface 133 of the dosage member part 132 seal shut the second end of the pressure release bore 123 during an initial part of the movement of the dosage member 130 from the load position to the delivery position, and progressively increasing portions of the pressure release bore 123, the dosage chamber 131 and the injection inlet 117 move into alignment during a final part of the movement of the dosage member 130 from the load to the delivery position.

When (1) the dosage member 130 is positioned at the load position so that powdered medicament 12 from the medicament reservoir 111 flows into the dosage chamber 131, (2) the pressure chamber is pressurized, and (3) the dosage member 130 is then moved from the load position to the del ivery position, pressurized gas will pass from the pressure chamber 122 through the pressure release bore 123 and discharge the powdered medicament 12 from the dosage chamber 131 into the medicament release bore 115.

The cartridge assembly 100 may optionally include a counter assembly 140 mounted within the counter assembly receiving cavity 116 for calculating the number of times the actuation arm 112 moves the dosage member 130 from the load position to the delivery position and back to the load position. Such a calculation affords an estimate of the number of dosages of medicament remaining in the medicament reservoir 111.

The counter assembly 140 comprises the actuation arm 112 having a counter assembly drive rod 141, the cartridge housing 114 having indicating means such as numerals (not shown) on its outer surfaces, a restraining assembly 142 comprising a leaf pawl 143 mounted within the counter assembly receiving cavity 116.

The counter assembly further includes the cartridge housing 114 having a ratchet wheel hub 144 (e.g. disposed on the restraining assembly 142), a ratchet wheel 138 having an axis, axially centered hub bearing surfaces 145, and a plurality of teeth 146 at its periphery. For example, the ratchet wheel 138 has a diameter of about 13.41 millimeters.

Each of the teeth 146 have a shoulder surface 147 and a release surface 148. The ratchet wheel bearing surfaces 145 are journaled on the ratchet wheel hub 144, and the ratchet wheel 138 is mounted within the counter assembly receiving cavity 116 for rotation relative to the cartridge housing 114. When the dosage member 130 moves from the delivery position to the load position, the drive rod 141 engages a shoulder surf ace 147 of a ratchet wheel tooth 146 to sequentially move the ratchet wheel 138 in a first rotational direction 137 (Figure 10) relative to the cartridge housing 114 to record the delivery of a dosage of medicament, and engagement between the leaf pawl 143 and a shoulder surface 147 of a ratchet wheel tooth 146 arrests movement of the rachet wheel 138 relative to the cartridge housing 114 in a direction opposite to the first rotational direction 137 when the drive rod 141 moves out of engagement with the shoulder surface 147 and along a release surface 148 of another ratchet wheel tooth 146 when the dosage member 130 moves from the load to the delivery position.

The counter assembly 140 may comprise only the rachet wheel 138 described above, when for example there are only a few dosages of medicament within the reservoir 111. In this example, the ratchet wheel 138 includes indicia such as an arrow (not shown) thereon for cooperating with the numerals (not shown) on the outer surface 119 of the housing to calculate the number of times the actuation arm 112 moves the dosage member 130 from the load position to the delivery position and back to the load position. However, preferably the counter assembly further comprises reduction means, particularly when the medicament reservoir 111 holds a large number of dosages (e.g. over 25).

The following described reduction means is believed to afford counting of up to about 210 dosages of medicament. The reduction means comprises the ratchet wheel 138 having an axially offset drive rib 150, an eccentric orbital gear 151 having an axis and bearing surfaces 152 adapted to receive the drive rib 150, and radially outwardly extending gear teeth 153. For example, the ratchet wheel 138 is constructed from any suitable material such as a polycarbonate or an acetal or an acetate, and has a pitch diameter of about 0.536 cm (0.211 inches).

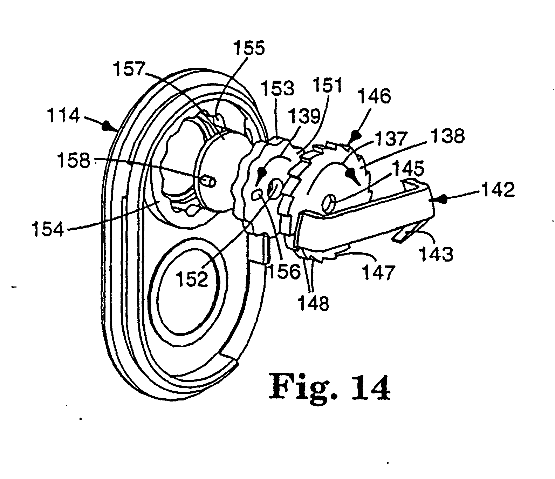

The reduction means also comprises the inner surfaces of the cartridge housing 114 having an annulus 154 (Figure 14) including an annulus axis, and radially inwardly extending gear teeth 155 for engaging the gear teeth 153 of the eccentric orbital gear 151. For example, the pitch diameter of the annulus 154 is approximately 0.58 cm (0.23 inches).

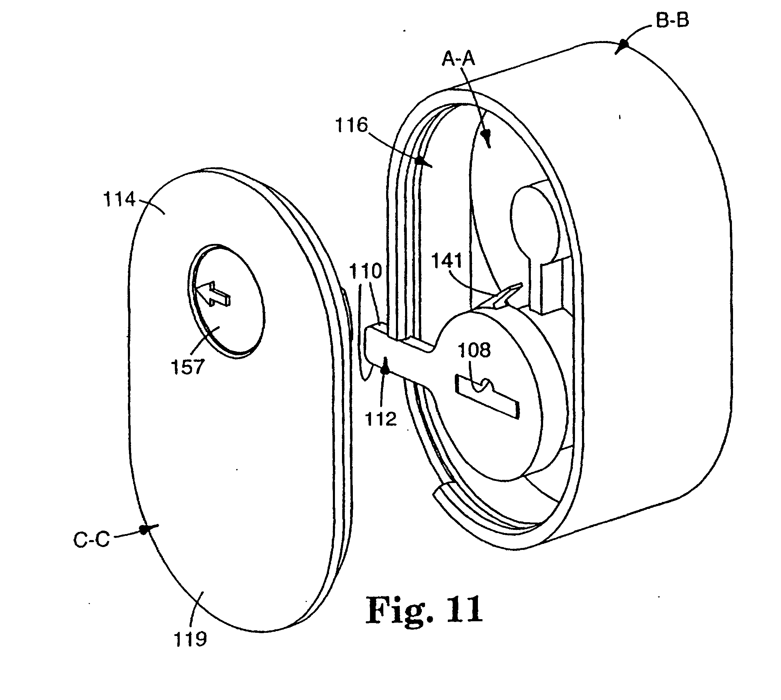

When the ratchet wheel 138 is driven in the first direction 137, the eccentric orbital gear teeth 153 engage the annulus gear teeth 155, and the eccentric orbital gear 151 moves in a second rotational direction 139 (Figure 10) generally opposite the first rotational direction 137. Optionally, the reduction means further includes the eccentric orbital gear 151 having an axially offset drive bearing slot surfaces 156, and a cover plate 157 having indicating indicia means (e.g. the arrow shown in Figures 10 and 11) thereon, and a drive finger 158 received in the bearing slot surfaces 156 of the eccentric orbital gear 151. In operation, when the eccentric orbital gear 151 moves relative to the annulus 154, the bearing slot surfaces 156 move the cover plate 157 in generally the second rotational direction 139 to thereby move the indicating indicia means (e.g. the arrow) on the cover plate 157 relative to the indicating means (e.g. the numerals not shown) on the outer surfaces 119 of the cartridge housing 114.

The bearing slot surfaces 156 are larger than the drive finger 158, and the drive finger may move within the slot surfaces 156. Thus, the cover plate 157 translates the eccentric motion of the eccentric orbital gear 151 into increments of generally circular motion so that the indicia means (e.g. the arrow) rotates in a defined, aesthetically pleasing circular path relative to the indicating means (e.g. the numerals) on the outer surfaces 119 of the cartridge housing 114.

The cartridge 100 also includes a powder loading assembly similar to the powder loading assembly for the dispenser 10 described above. The cartridge 100 and powder loading assembly afford storage of powdered medicament 12 in a reservoir 111 that (1) may be stirred or agitated after the cartridge 100 is delivered to the ultimate user, and (2) may be stored remote from the dispenser 200 prior to its use.

The powder loading assembly is a means for transferring dry powder medicament from the medicament reservoir 111 to the dosage chamber 131 in the dosage member 130 when the dosage member 130 is in the load position.

The powder loading assembly comprises at least one and preferably several flexible blades 160 having proximal and distal ends. The blades 160 may be constructed, for example, from a polyetherimid material such as grade #1000 ULTEM generally available from General Electric of Pittsfield, Massachusetts. Like the blade 32 of the dispenser 10, the blade 160 separates agglomerates of the dry powder medicament 12 into smaller agglomerates and separates the dry powder from walls of the medicament reservoir 111.

The powder loading assembly also includes a blade shaft 161 connected to the blade 160, and a one-way gear clutch 162 connected to the blade shaft 161 and adapted to be engaged by the actuation means 210 of the medicament dispenser 200. For example, the actuation means of the medicament dispenser 200 may include a complementary spring loaded gear clutch 275 which engages the one-way gear clutch 162 to drive the blade(s) 160 in a predetermined powder loading direction, and which releases from the one-way gear clutch 162 when the spring loaded gear clutch 275 is rotated in a direction opposite the powder loading direction.

Also like the blade 36 in the dispenser 10, when the blade 160 in cartridge 100 is mounted within the medicament reservoir and is driven by the actuation means 210 of the medicament dispenser 200, the blade 160 moves along first and second predetermined paths within the medicament reservoir 111. During a first part of the predetermined path, the leading surface of the blade 160 leads and the distal end of the blade 160 moves along a portion of the inner surface defining the medicament reservoir 111. During a second portion of the predetermined path, the distal end of the blade 160 moves along and contacts a portion of the outer sealing surface 133 of the dosage member 130 to progressively increasingly bend the blade 160 to form the leading surface into a convex surface and to move the leading surface progressively closer to the portion of the outer sealing surface 133 of the dosage member 130 adjacent the dosage chamber 131 to pack powdered medicament into the dosage chamber 131 (see Figure 7A).

### OPERATION OF THE CARTRIDGE

The operation of the cartridge according to the present invention will now be described with reference to the preferred embodiment 100 and with reference to an example of a dispenser 200. Figures 20 and 21 sequentially illustrate the operation of the cartridge 100 in conjunction with the dispenser 200.

The cartridge 100 shown in Figures 20 and 21 is generally identical to the cartridge 100 shown in Figures 10 through 19 except that the position of the actuation arm 112 in the load and delivery positions is generally lower in Figures 20 and 21 than the position of the actuation arm 112 in Figures 10 through 19. The actuation arm 112 is shown in this manner to better illustrate the operation of the actuation means 210 of the dispenser 200.

The cartridge 100 is received in the dispenser 200 which includes a housing 202 including a mouthpiece portion 201, pressurization means 211 and actuation means 210. The actuation means is preferably a mechanical assembly which minimizes or reduces the inputs or operations required from a user. However, it should be noted that the invention comprises the cartridge 100 and thus, the actuation means may comprise a variety of different actuation means from the strictly manual actuation similar to that described with respect to the dispenser 10 to completely automatic actuation means or combinations of manual and automatic actuation means.

The actuation means 210 includes, for example, the actuation means shown in Figures 20 and 21. Those actuation means include a cammed fork member 204 (shown in Figures 20 and 21 by dashed lines) having surfaces 206 adapted to receive the manipulation surfaces 110 of the actuation arm 112. The cammed fork member 204 is pivotally mounted on the housing 202 of the dispenser 200 to move between a load (Figure 21) and delivery position (Figure 20) corresponding to the positions of the actuation arm 112.

The actuation means 210 includes rack 220 and gear 221 assemblies. The rack 220 includes a button member 219 that is manually slidable within guide surfaces of housing 202 between an "armed" position shown in Figure 21 and a release position shown in Figure 20. When the button member 219 is slid to the armed position, the gear 221 rotates clockwise in the drawing and causes a piston member in the pressurization means 211 to compress fluid (e.g. air) within the pressure chamber 122 of the cartridge. Alternatively, the rack 220/button member 219 may be replaced with a circular gear (not shown) connected to and driven by a pivotal mouthpiece cover (not shown).

Clockwise rotation of the gear 221 in Figure 20 also causes gear 276 to rotate spring drive clutch 275. Spring drive clutch 275 engages one-way gear clutch 162 to drive the blades 160 within the medicament reservoir 111 in a predetermined powder loading direction and releases and does not drive the one-way gear clutch 162 when the gear 221 rotates counter-clockwise in Figure 20.

At generally the same time that the button member 219 is slid to the armed position, a linkage 225 causes the cammed fork member 204 to pivot about its pivot point on housing 202 against the bias of firing spring 226. The linkage 225 will cause the cammed fork member 204 to pivot until just after the fork member 204 engages cam surfaces 231 on latch member 230. The latch member 230 is pivotally mounted on the housing 202 for movement between a latched and release position. The latch member 230 includes a latching spring 235 for biasing the latch member 230 toward the latched position, and a release button 238 for manually pivoting the latch member 230 against the bias of the spring 235.

When the cammed fork member 204 engages cam surfaces 231, the latch member 230 pivots against the bias of spring 235 out of the path of the cammed fork member 204 until a trailing edge of the cammed fork member 204 clears the cam surfaces 231 then engages shoulder surfaces 239 of latch 230. This is the position of the cammed fork member 204 and the latch 230 shown in Figure 21.

The linkage 225 causes the actuation arm 112 to move from the delivery to the load position before the button 219 moves completely to the armed position. During a final portion of the movement of the button 219 to the armed position, the actuation arm 112 will be in the delivery position and the spring drive clutch 275 will cause the blades 160 to load the dosage chamber 131 with medicament.

After the dosage chamber 131 is loaded with a dosage of medicament and after the actuation arm 112/cammed fork member 204 is in the position shown in Figure 21, the dispenser is ready for actuation. To actuate the dispenser 200, a user manually presses on the button 238 which releases cammed fork member 204 which is under the bias of spring 226. The spring 226 moves the cammed fork member 204 from the load to the delivery position and consequently the actuation arm 112 from the load to the delivery position. It should be noted that the latch member 230 need not be a button member but may instead comprise a breath actuated means such as the breath actuated means shown in U.S. Patents 5,069,204; 4,664,107; and those mentioned in 4,664,107 including 3,187,748; 3,456,644; 3,645,645; 3,456,646; 3,565,070; 3,598,294; 3,814,297; 3,605,738; 3,732,864; 3,636,949; 3,789,843 and 3,187,748.

Referring now to Figure 21, the linkage 225 comprise a slider member at its distal end. The linkage 225 may be constructed to move the gear 221 and the button 219 back to the position shown in Figure 20 after the button 238 is pressed. Alternatively, the linkage 225 may be constructed to release after the button 238 is pressed. Instead, the user may manually move the button 219 from the position shown in Figure 21 to the position shown in Figure 20 after the cartridge 100 is fired.

## Claims

1. A dry powder medicament dispenser (10) for the delivery of a dosage unit of drug comprising:
(A) a mouthpiece portion (15) adapted to be positioned in a patient's mouth;
(B) movable chamber means (32) capable of receiving and containing an agglomerated mass of micronized particles of a drug having an average particle diameter of from about 0.3 micrometers to about 20 micrometers;
(C) pressurized chamber means (22) having an opening (23) which is adapted to be intermittently closed to the atmosphere, said pressurized chamber means (22) being capable of containing a supply of pressurized air at least when said opening (23) is closed to the atmosphere;
(D) said movable chamber means (32) being movable from a first, medicament receiving, position to a second, medicament discharge, position, said movable chamber means (32) being immediately adjacent and juxtaposed with said opening (23) when in said second position; and
(E) said supply of pressurized air being immediately adjacent the position of said movable chamber means (32) when said movable chamber means (32) is in said second position, whereby the dispenser (10) is free of an unpressurized region of air between said supply of pressurized air and said movable chamber means (32);
whereby when said movable chamber means (32) is in said second position there is an immediate release of said pressurized air to said agglomerated mass, thereby disintegrating the agglomerated mass into said plurality of micronized particles for delivery to said user.

2. A dispenser (10) further comprising:
a housing (14),
means for providing a packed, predetermined, agglomerated dose of the dry powder medicament (12) in a said chamber means (32), and
an internal pressure generator (21) for generating a pressure for forcibly expelling the predetermined dose from the chamber means (32) and external of said housing (14) in micronized, deagglomerated form suitable for inhalation therapy.

3. A dispenser (10) according to claim 2 wherein the pressure generator (21) suddenly provides a fluid pressure differential across the packed dose so that the fluid flows to deagglomerate the packed dose into a plurality of respirable particles.

4. A dispenser (10) according to claim 3 wherein the fluid pressure differential is a substantial fluid pressure differential.

5. A dispenser (10) according to any of claims 2-4, wherein the pressure generator (21) comprises a pressure chamber (22) for providing a first fluid pressure which is greater than ambient pressure,
the dose is situated adjacent ambient pressure so that release of the first pressure provides a pressure differential across the packed dose and a fluid flow which deagglomerates the dose, and
wherein the pressure differential provided across the dose is the maximum pressure differential that may be provided with the first pressure and ambient pressure.

6. A dispenser (10) according to any of claims 2-5, wherein the pressure generator (21) provides a fluid flow at the dose from fluid which is initially free of velocity but which reaches a maximum velocity soon after the fluid begins to flow.

7. A dispenser (10) according to any one of claims 2 to 5 wherein the pressure generator (21) provides a fluid flow from fluid which is initially free of velocity but which subsequently comprises a high velocity turbulent fluid flow relative to the packed dose that deagglomerates the packed dose into a plurality of respirable particles.

8. A dispenser (10) according to claim 7 wherein the high velocity turbulent fluid flow is provided soon after the fluid begins to flow.

9. A dispenser (10) according to any of claims 2-8, wherein the pressure generator (21) provides a fluid pressure differential to progressively increasing portions of the dose.

10. A dispenser (10) according to any one of claims 2 to 8 wherein the pressure generator (21) provides a pressure differential across the dose by releasing a pressurized fluid generally immediately adjacent the dose.

11. A dispenser (10) according to any one of the preceding claims, wherein said particle diameters are between 0.5 micrometers and 6 micrometers.

12. A dispenser (10) according to any of claims 2-11, wherein the dispenser comprises a dosage member (30) having surfaces defining the chamber means (32), wherein the chamber means (32) has a longitudinal axis (f) and extends through the dosage member (30) between spaced parts of an outer sealing surface (33), and
the means for providing a packed, predetermined agglomerated dose comprises:
a medicament reservoir (11) for storing the drug, and
an agglomerator within the medicament reservoir (11) for providing a positive, orientation independent packing force for loading said dry powder medicament (12) from said medicament reservoir (11) into said dosage chamber (32) under pressure sufficient to pack the dry powder medicament into a reproducible dose.

13. A dispenser (10) according to claim 12 wherein the agglomerator is mounted for movement across the dosage chamber (32) so that the positive, orientation independent packing force has a component that is generally parallel to the longitudinal axis (f).

14. A dispenser (10) according to claim 13 wherein the component of the packing force that is generally parallel to the longitudinal axis (f) progressively increases as the agglomerator moves across at least a portion of the dosage chamber (32).

15. A dispenser (10) according to claim 14 wherein the agglomerator comprises a blade (36) which progressively increasingly bends as it moves across the dosage chamber (32).

16. A dispenser (10) according to any of the preceding claims wherein the dispenser (10) has an effectiveness in deagglomerating the packed dose which is generally independent of user inhalation rate.

17. A dispenser (10) according to any of claims 1 to 16 wherein between the load and delivery orientations, progressively increasing portions of the dosage chamber (32), medicament delivery passageway (16) and pressure outlet (23) move into alignment.

18. Use of the dispenser according to any one of the preceding claims for the delivery of a dosage unit of drug comprising the steps of:
(D) moving said movable chamber means (32) from a first, medicament receiving, position to a second, medicament discharge, position, said movable chamber means (32) being immediately adjacent and juxtaposed with said opening (23) when in said second position; wherein.
(E) said supply of pressurized air is immediately adjacent the position of said movable chamber means (32) when said movable chamber means (32) is in said second position, whereby the dispenser (10) is free of an unpressurized region of air between said supply of pressurized air and said movable chamber means (32);
whereby there is an immediate release of said pressurized air to said agglomerated mass, thereby disintegrating the agglomerated mass into said plurality of micronized particles for delivery to said user.

19. Use of claim 18 further comprising:
providing micronized particles of the medicament within a housing (14),
packing the micronized particles into a predetermined, agglomerated dose in the chamber means (32), and
generating a fluid pressure to forcibly expel the predetermined dose from the chamber means (32) externally of said housing (14) in micronized deagglomerated form suitable for inhalation therapy.

20. Use according to claim 19 wherein the step of generating a fluid pressure comprises the step of:
suddenly generating a substantial fluid pressure differential across the packed dose resulting in fluid flow which deagglomerates the dose into a plurality of respirable particles.

21. Use according to claim 19 wherein the step of generating a fluid pressure comprises the step of :
generating a fluid flow adjacent the packed dose from fluid which is initially generally free of velocity such that the fluid flow subsequently reaches a maximum velocity soon after the fluid begins to flow.

22. Use according to claim 19 wherein the step of generating a fluid pressure comprises the step of:
generating a fluid flow comprising a high velocity turbulent fluid flow relative to the packed dose that deagglomerates the packed dose into a plurality of respirable particles.

23. Use according to claim 22 wherein the high velocity turbulent fluid flow is provided soon after the fluid begins to flow.

24. Use according to claim 19 wherein said steps of providing micronized particles of the medicament (12) within a housing (14) and packing the micronized particles into a predetermined, agglomerated dose in a dosage chamber (32) comprise the steps of:
providing a dosage member (30) having surfaces defining the dosage chamber (32), wherein the dosage chamber (32) has a longitudinal axis (f) and extends through the dosage member (30) between spaced parts of an outer sealing surface (33), and
storing a supply of dry powder medicament (12) in a medicament reservoir (11),
using an agglomerator within the medicament reservoir (11) that provides a positive, orientation independent packing force for loading the dry powder medicament (12) from the medicament reservoir (11) into the dosage chamber (32) under pressure sufficient to pack the dry powder medicament into a reproducible dose.

25. Use according to claim 24 wherein
the agglomerator moves across the dosage chamber (32) so that the positive, orientation independent packing force has a component that is generally parallel to the longitudinal axis (f).

26. Use according to claim 25 further comprising the step of progressively increasing the component of the packing force that is generally parallel to the longitudinal axis (f) as the agglomerator moves across at least a portion of the dosage chamber (32).

27. Use according to claim 19 wherein the step of generating a fluid pressure comprises the step of releasing a fluid pressure generally immediately adjacent the dose.

28. Use according to claim 19 wherein the step of generating a fluid pressure comprises the steps of:
providing a first pressure which is greater than ambient pressure,
situating the dose adjacent ambient pressure,
releasing the first pressure to provide a pressure differential across the packed dose so that fluid will flow to deagglomerate the dose, and
wherein the pressure differential provided across the dose is the maximum pressure differential that may be provided with the first pressure and ambient pressure.

## Patentansprüche

1. Trockenpulvermedikamentenspender (10) zur Abgabe einer Dosiseinheit eines Arzneimittels mit:
(A) einem Mundstückabschnitt (15), der ausgebildet ist, um im Mund eines Patienten angeordnet zu werden;
(B) einer bewegbaren Kammereinrichtung (32), die in der Lage ist, eine agglomerierte Masse von mikronisierten Partikeln eines Medikaments, die einen durchschnittlichen Partikeldurchmesser von ungefähr 0,3 Mikrometer bis ungefähr 20 Mikrometer haben, zu empfangen und zu halten;
(C) einer unter Druck stehenden Kammereinrichtung (22) mit einer Öffnung (23), die eingerichtet ist, um zeitweise zur Atmosphäre hin geschlossen zu werden, wobei die unter Druck stehende Kammer (22) in der Lage ist, einen Vorrat Druckluft mindestens dann zu halten, wenn die Öffnung (23) zur Atmosphäre hin geschlossen ist;
(D) wobei die bewegbare Kammereinrichtung (32) von einer ersten medikamentaufnehmenden Position in eine zweite medikamentabgebende Position bewegbar ist, wobei in der zweiten Position die bewegbare Kammereinrichtung (32) unmittelbar an und neben der Öffnung (23) angeordnet ist; und
(E) wobei der Druckluftvorrat unmittelbar neben der Position der bewegbaren Kammereinrichtung (32) ist, wenn die bewegbare Kammereinrichtung (32) in der zweiten Position ist, wodurch der Spender (10) zwischen dem Druckluftvorrat und der bewegbaren Kammereinrichtung (32) keinen Luftbereich hat, der nicht unter Druck steht;
wobei, wenn die bewegbare Kammereinrichtung (32) in der zweiten Position ist, es eine unmittelbare Freisetzung der Druckluft zur agglomerierten Masse gibt, wodurch die agglomerierte Masse in die Mehrzahl mikronisierter Partikel zerfällt, um an den Benutzer abgegeben zu werden.

2. Spender (10), ferner mit:
einem Gehäuse (14);
einer Einrichtung zur Bereitstellung einer dichtgepackten vorbestimmten agglomerierten Dosis des Trockenpulvermedikaments (12) in der Kammereinrichtung (32); und
einem inneren Druckgenerator (21) zum Erzeugen eines Drucks, um die vorgegebene Dosis in einer für eine Inhalationstherapie geeigneten, mikronisierten, deagglomerierten Form kraftvoll aus der Kammereinrichtung (32) und aus dem Gehäuse (14) auszustoßen.

3. Spender (10) nach Anspruch 2, wobei der Druckgenerator (21) plötzlich einen Fluiddruckunterschied über die dichtgepackte Dosis hinweg bereitstellt, so daß das Fluid strömt, um die dichtgepackte Dosis in eine Mehrzahl einatembare Partikel zu deagglomerieren.

4. Spender (10) nach Anspruch 3, wobei der Fluiddruckunterschied ein wesentlicher Fluiddruckunterschied ist.

5. Spender (10) nach einem der Ansprüche 2 bis 4, wobei der Druckgenerator (21) eine Druckkammer (22) zur Bereitstellung eines ersten Fluiddrucks aufweist, der größer als der Umgebungsdruck ist,
die Dosis neben dem Umgebungsdruck angeordnet ist, so daß ein Freisetzen des ersten Drucks einen Druckunterschied über die dichtgepackte Dosis hinweg und einen Fluidstrom bereitstellt, der die Dosis deagglomeriert, und
wobei der über die Dosis bereitgestellte Druckunterschied der maximale Druckunterschied ist, der mit dem ersten Druck und dem Umgebungsdruck bereitgestellt werden kann.

6. Spender (10) nach einem der Ansprüche 2 bis 5, wobei der Druckgenerator (21) an der Dosis einen Fluidstrom aus einem Fluid bereitstellt, das anfänglich keine Geschwindigkeit hat, das aber eine maximale Geschwindigkeit erreicht, bald nachdem das Fluid zu strömen begonnen hat.

7. Spender (10) nach einem der Ansprüche 2 bis 5, wobei der Druckgenerator (21) einen Fluidstrom aus einem Fluid bereitstellt, das anfänglich keine Geschwindigkeit hat, das aber anschließend einen turbulenten Fluidstrom hoher Geschwindigkeit relativ zur dichtgepackten Dosis aufweist, der die dichtgepackte Dosis in eine Mehrzahl einatembarer Partikel deagglomeriert.

8. Spender (10) nach Anspruch 7, wobei der turbulente Fluidstrom hoher Geschwindigkeit bereitgestellt wird, bald nachdem das Fluid zu strömen begonnen hat.

9. Spender (10) nach einem der Ansprüche 2 bis 8, wobei der Druckgenerator (21) einen Fluiddruckunterschied an immer mehr Abschnitten der Dosis bereitstellt.

10. Spender (10) nach einem der Ansprüche 2 bis 8, wobei der Druckgenerator (21) durch Freisetzen eines unter Druck stehenden Fluids im allgemeinen unmittelbar neben der Dosis einen Druckunterschied über die Dosis hinweg bereitstellt.

11. Spender (10) nach einem der vorstehenden Ansprüche,
wobei die Partikeldurchmesser zwischen 0,5 Mikrometer und 6 Mikrometer sind.

12. Spender (10) nach einem der Ansprüche 2 bis 11, wobei der Spender ein Dosierelement (30) aufweist, das Oberflächen hat, die die Kammereinrichtung (32) umgrenzen, wobei die Kammereinrichtung (32) eine Längsachse (f) hat und sich zwischen beabstandeten Teilen einer äußeren Abdichtungsfläche (33) durch das Dosierelement (30) hindurch erstreckt, und die Einrichtung zur Bereitstellung einer dichtgepackten vorgegebenen agglomerierten Dosis aufweist:
ein Medikamentenreservoir (11) zum Aufbewahren des Arzneimittels, und
einen Agglomerator innerhalb des Medikamentenreservoirs (11) zum Bereitstellen einer positiven richtungsunabhängigen Verdichtungskraft zum Laden des Trockenpulvermedikaments (12) aus dem Medikamentenreservoir (11) in die Dosierkammer (32) unter einem Druck, der ausreichend ist, um das Trockenpulvermedikament in eine reproduzierbare Dosis zu packen.

13. Spender (10) nach Anspruch 12, wobei der Agglomerator für eine Bewegung über die Dosierkammer (32) hinweg angebracht ist, so daß die positive, richtungsunabhängige Verdichtungskraft eine Komponente hat, die im allgemeinen parallel zur Längsachse (f) ist.

14. Spender (10) nach Anspruch 13, wobei sich diejenige Komponente der Verdichtungskraft, die im allgemeinen parallel zur Längsachse (f) ist, fortlaufend vergrößert, während sich der Agglomerator über mindestens einen Abschnitt der Dosierkammer (32) hinweg bewegt.

15. Spender (10) nach Anspruch 14, wobei der Agglomerator ein Blatt (36) aufweist, das sich immer mehr biegt, während es sich über die Dosierkammer (32) hinweg bewegt.

16. Spender (10) nach einem der vorstehenden Ansprüche, wobei die Leistungsfähigkeit des Spenders (10) die dichtgepackte Dosis zu deagglomerieren, im allgemeinen unabhängig von der Inhalationsrate des Benutzers ist.

17. Spender (10) nach einem der Ansprüche 1 bis 16, wobei sich zwischen der Lade- und der Abgabeorientierung immer mehr Abschnitte der Dosierkammer (32), des Medikamentenabgabekanals (16) und des Druckauslasses (23) in eine gemeinsame Ausrichtung bewegen.

18. Verwendung des Trockenpulvermedikamentenspenders nach einem der vorstehenden Ansprüche zur Abgabe einer Arzneimitteldosiseinheit, mit den folgenden Schritten:
(D) Bewegen der bewegbaren Kammereinrichtung (32) von einer ersten medikamentaufnehmenden Position in eine zweite medikamentabgebende Position, wobei in der zweiten Position die bewegbare Kammereinrichtung (32) unmittelbar an und neben der Öffnung (23) angeordnet ist; wobei
(E) der Druckluftvorrat unmittelbar neben der Position der bewegbaren Kammereinrichtung (32) ist, wenn die bewegbare Kammereinrichtung (32) in der zweiten Position ist, wobei der Spender (10) zwischen dem Druckluftvorrat und der bewegbaren Kammereinrichtung (32) keinen Luftbereich hat, der nicht unter Druck steht;
wobei es eine unmittelbare Freisetzung der Druckluft zur agglomerierten Masse gibt, wodurch die agglomerierte Masse in die Mehrzahl mikronisierter Partikel zerfällt, um an den Benutzer abgegeben zu werden.

19. Verwendung nach Anspruch 18, ferner mit:
Bereitstellen von mikronisierten Partikeln des Medikaments in einem Gehäuse (14),
Verdichten der mikronisierten Partikel in eine vorgegebene agglomerierte Dosis in der Kammereinrichtung (32), und
Erzeugen eines Fluiddrucks, um die vorgegebene Dosis in einer für eine Inhalationstherapie geeigneten, mikronisierten, deagglomerierten Form kraftvoll aus der Kammereinrichtung (32) und aus dem Gehäuse (14) auszustoßen.

20. Verwendung nach Anspruch 19, wobei der Schritt der Erzeugung eines Fluiddrucks den folgenden Schritt aufweist:
plötzliches Erzeugen eines wesentlichen Fluiddruckunterschieds über die dichtgepackte Dosis hinweg, wodurch sich ein Fluidstrom ergibt, der die Dosis in eine Mehrzahl einatembarer Partikel deagglomeriert.

21. Verwendung nach Anspruch 19, wobei der Schritt der Erzeugung eines Fluiddrucks den folgenden Schritt aufweist:
Erzeugen eines Fluidstroms, neben der dichtgepackten Dosis, aus einem Fluid, das anfangs keine Geschwindigkeit hat, so daß der Fluidstrom anschließend eine maximale Geschwindigkeit erreicht, bald nachdem das Fluid zu strömen begonnen hat.

22. Verwendung nach Anspruch 19, wobei der Schritt der Erzeugung eines Fluiddrucks den folgenden Schritt aufweist:
Erzeugen eines Fluidstroms, der einen turbulenten Fluidstrom hoher Geschwindigkeit relativ zur dichtgepackten Dosis aufweist, der die dichtgepackte Dosis in eine Mehrzahl einatembarer Partikel deagglomeriert.

23. Verwendung nach Anspruch 22, wobei der turbulente Fluidstrom hoher Geschwindigkeit bereitgestellt wird, bald nachdem das Fluid zu strömen begonnen hat.

24. Verwendung nach Anspruch 19, wobei der Schritt der Bereitstellung von mikronisierten Partikeln des Medikaments (12) innerhalb eines Gehäuses (14) und der Schritt des Verdichtens der mikronisierten Partikel in eine vorgegebene agglomerierte Dosis in einer Dosierkammer (32) die folgenden Schritte aufweist:
Bereitstellen eines Dosierelements (30), das Oberflächen hat, die die Dosierkammer (32) umgrenzen, wobei die Dosierkammer (32) eine Längsachse (f) hat und sich zwischen beabstandeten Teilen einer äußeren Abdichtungsfläche (33) durch das Dosierelement (30) hindurch erstreckt, und
Aufbewahren eines Vorrats Trockenpulvermedikament (12) in einem Medikamentenreservoir (11),
Verwenden eines Agglomerators innerhalb des Medikamentenreservoirs (11), der eine positive, richtungsunabhängige Verdichtungskraft bereitstellt, um das Trockenpulvermedikament (12) unter einem Druck, der ausreichend ist, um das Trockenpulvermedikament in eine reproduzierbare Dosis zu verdichten, vom Medikamentenreservoir (11) aus in die Dosierkammer (32) zu laden.

25. Verwendung nach Anspruch 24, wobei sich der Agglomerator über die Dosierkammer (32) hinweg bewegt, so daß die positive richtungsunabhängige Verdichtungskraft eine Komponente hat, die im allgemeinen parallel zur Längsachse (f) ist.

26. Verwendung nach Anspruch 25, ferner mit dem Schritt der fortlaufenden Verstärkung derjenigen Komponente der Verdichtungskraft, die im allgemeinen parallel zur Längsachse (f) ist, während sich der Agglomerator über mindestens einen Abschnitt der Dosierkammer (32) hinweg bewegt.

27. Verwendung nach Anspruch 19, wobei der Schritt der Erzeugung eines Fluiddrucks den Schritt des Freisetzens eines Fluiddrucks im allgemeinen unmittelbar neben der Dosis aufweist.

28. Verwendung nach Anspruch 19, wobei der Schritt der Erzeugung eines Fluiddrucks die folgenden Schritte aufweist:
Bereitstellen eines ersten Drucks, der größer als der Umgebungsdruck ist,
Anordnen der Dosis neben dem Umgebungsdruck,
Freisetzen des ersten Drucks, um einen Druckunterschied über die dichtgepackte Dosis hinweg bereitzustellen, so daß Fluid strömen wird, um die Dosis zu deagglomerieren, und
wobei der über die Dosis hinweg bereitgestellte Druckunterschied der maximale Druckunterschied ist, der mit dem ersten Druck und dem Umgebungsdruck bereitgestellt werden kann.

## Revendications

1. Distributeur de médicament en poudre sèche (10) adapté pour la distribution d'une unité de dosage de médicament comprenant :
(A) une partie d'embout (15) adaptée pour être logée dans la bouche d'un patient ;
(B) des moyens de chambre mobile (32) capables de recevoir et de conserver une masse agglomérée de particules micronisées d'un médicament ayant un diamètre de particule moyen compris entre 0,3 micromètre environ et 20 micromètres environ ;
(C) des moyens de chambre pressurisés (22) comportant une ouverture (23) qui est adaptée pour être fermée par intermittence par rapport à l'atmosphère ; lesdits moyens de chambre pressurisés (22) étant capables de conserver une alimentation en air sous pression au moins lorsque ladite ouverture (23) est fermée par rapport à l'atmosphère ;
(D) lesdits moyens de chambre mobile (32) étant susceptibles d'être déplacés depuis une première position de réception du médicament jusqu'à une deuxième position d'injection du médicament, lesdits moyens de chambre mobile (32) se trouvant immédiatement adjacents et juxtaposés à ladite ouverture (23) lorsqu'ils se trouvent dans ladite deuxième position ; et
(E) ladite alimentation en air sous pression se trouvant immédiatement adjacente à la position desdits moyens de chambre mobile (32) lorsque lesdits moyens de chambre mobile (32) se trouvent dans ladite deuxième position ; moyennant quoi le distributeur (10) est exempt d'une région non soumise à une pression de l'air entre ladite alimentation en air sous pression et lesdits moyens de chambre mobile (32) ;
moyennant quoi lorsque lesdits moyens de chambre mobile (32) se trouvent dans ladite deuxième position, il se produit une injection immédiate dudit air sous pression en direction de ladite masse agglomérée, qui provoque la désintégration de la masse agglomérée en ladite pluralité de particules micronisées qui peuvent alors être distribuées au dit utilisateur.

2. Distributeur (10) comprenant en outre :
une enveloppe (14) ;
des moyens adaptés pour distribuer une dose concentrée, agglomérée, prédéterminée, du médicament en poudre sèche (12) dans lesdits moyens de chambre (32) ; et
un dispositif de génération de pression interne (21) adapté pour produire une pression adaptée pour expulser par la force la dose prédéterminée à partir des moyens de chambre (32) et à l'extérieur de ladite enveloppe (14) sous une forme micronisée, désintégrée, adaptée pour une thérapie par inhalation.

3. Distributeur (10) selon la revendication 2, dans lequel le dispositif de génération de pression (21) injecte subitement une pression de fluide différentielle à travers la dose concentrée de sorte que le fluide s'écoule afin de désintégrer la dose concentrée en une pluralité de particules respirables.

4. Distributeur (10) selon la revendication 3, dans lequel la pression de fluide différentielle est une pression de fluide différentielle substantielle.

5. Distributeur (10) selon l'une quelconque des revendications 2 à 4, dans lequel le dispositif de génération de pression (21) comprend une chambre sous pression
(22) adaptée pour produire une première, pression de fluide qui est plus élevée qu'une pression ambiante ;
la dose est située adjacente à une pression ambiante de sorte que l'injection de la première pression fournit une pression différentielle à travers la dose concentrée et un écoulement de fluide qui vient désintégrer la dose ; et
dans lequel une pression différentielle fournie à travers la dose correspond à une pression différentielle maximale susceptible d'être produite avec la première pression et la pression ambiante.

6. Distributeur (10) selon l'une quelconque des revendications 2 à 5, dans lequel le dispositif de génération de pression (21) fournit un écoulement de fluide au niveau de la dose à partir du fluide qui est initialement exempt de toute vitesse mais qui atteint une vitesse maximale rapidement après que le fluide a commencé de s'écouler.

7. Distributeur (10) selon l'une quelconque des revendications 2 à 5, dans lequel le dispositif de génération de pression (21) fournit un écoulement de fluide à partir du fluide qui est initialement exempt de toute vitesse mais qui par la suite se transforme en un écoulement de fluide turbulent et très rapide par rapport à la dose concentrée, qui désintègre la dose concentrée en une pluralité de particules respirables.

8. Distributeur (10) selon la revendication 7, dans lequel l'écoulement de fluide turbulent et très rapide est fourni rapidement après que le fluide a commencé de s'écouler.

9. Distributeur (10) selon l'une quelconque des revendications 2 à 8, dans lequel le dispositif de génération de pression (21) fournit une pression de fluide différentielle à des quantités de la dose qui augmentent progressivement.

10. Distributeur (10) selon l'une quelconque des revendications 2 à 8, dans lequel le dispositif de génération de pression (21) fournit une pression différentielle à travers la dose en libérant un fluide sous pression généralement immédiatement adjacent à la dose.

11. Distributeur (10) selon l'une quelconque des revendications précédentes, dans lequel les diamètres desdites particules sont compris entre 0,5 micromètre et 6 micromètres.

12. Distributeur (10) selon l'une quelconque des revendications 2 à 11, dans lequel le distributeur comprend un organe de dosage (30) dont les surfaces définissent les moyens de chambre (32), dans lequel les moyens de chambre (32) ont un axe longitudinal (f) et s'étendent à travers l'organe de dosage (30) entre des parties espacées d'une surface jointive extérieure (33) ; et
les moyens adaptés pour fournir une dose concentrée agglomérée prédéterminée comprennent :
un réservoir à médicament (11) adapté pour conserver le médicament ; et
un dispositif d'agglomération situé à l'intérieur du réservoir à médicament (11) et adapté pour produire une force de compactage positive, indépendante de l'orientation qui permet de charger ledit médicament en poudre sèche (12) à partir dudit réservoir à médicament (11) dans lesdits moyens de chambre (32) sous une pression suffisante pour concentrer le médicament en poudre sèche et en faire une dose reproductible.

13. Distributeur (10) selon la revendication 12, dans lequel le dispositif d'agglomération est installé de manière à pouvoir se déplacer à travers les moyens de chambre (32) si bien que la force de compactage positive, indépendante de l'orientation a un composant qui est généralement parallèle à l'axe longitudinal (f).

14. Distributeur (10) selon la revendication 13, dans lequel le composant de la force de compactage qui est généralement parallèle à l'axe longitudinal (f) augmente progressivement à mesure que le dispositif d'agglomération se déplace à travers au moins une partie des moyens de chambre (32).

15. Distributeur (10) selon la revendication 14, dans lequel le dispositif d'agglomération comprend une lamelle (36) qui se courbe progressivement un peu plus à mesure qu'elle se déplace à travers les moyens de chambre (32).

16. Distributeur (10) selon l'une quelconque des revendications précédentes, dans lequel le distributeur (10) présente une efficacité adaptée pour désintégrer la dose concentrée qui est généralement indépendante de la vitesse d'inhalation de l'utilisateur.

17. Distributeur (10) selon l'une quelconque des revendications 1 à 16, dans lequel, entre les orientations de chargement et de distribution, des parties des moyens de chambre (32) qui augmentent progressivement, une voie de passage de distribution du médicament (16) et un passage de sortie à pression (23) se déplacent en alignement.

18. Procédé d'utilisation du distributeur selon l'une quelconque des revendications précédentes, adapté pour la distribution d'une unité de dosage de médicament, comprenant les étapes consistant à :
(D) déplacer lesdits moyens de chambre mobile (32) depuis une première position de réception du médicament jusqu'à une deuxième position d'injection du médicament, lesdits moyens de chambre mobile (32) se trouvant immédiatement adjacents et juxtaposés à ladite ouverture (23) lorsqu'ils se trouvent dans ladite deuxième position, dans lesquels :
(E) ladite alimentation en air sous pression se trouve immédiatement adjacente à la position desdits moyens de chambre mobile (32) lorsque lesdits moyens de chambre mobile (32) se trouvent dans ladite deuxième position ; moyennant quoi le distributeur (10) est exempt d'une région non soumise à une pression de l'air entre ladite alimentation en air sous pression et lesdits moyens de chambre mobile (32) ;
moyennant quoi il se produit une injection immédiate dudit air sous pression en direction de ladite masse agglomérée, qui provoque la désintégration de la masse agglomérée en ladite pluralité de particules micronisées qui peuvent alors être distribuées au dit utilisateur.

19. Procédé d'utilisation selon la revendication 18 comprenant en outre les étapes consistant à :
injecter des particules micronisées du médicament à l'intérieur d'une enveloppe (14) ;
concentrer les particules micronisées pour former une dose agglomérée prédéterminée dans les moyens de chambre (32) ; et
produire une pression de fluide adaptée pour expulser par la force la dose prédéterminée à partir des moyens de chambre (32) à l'extérieur de ladite enveloppe (14) sous une forme micronisée, désintégrée, adaptée pour une thérapie par inhalation.

20. Procédé d'utilisation selon la revendication 19, dans lequel l'étape consistant à produire une pression de fluide comprend l'étape consistant à :
produire subitement une pression de fluide différentielle substantielle à travers la dose concentrée de sorte que le fluide s'écoule afin de désintégrer la dose concentrée en une pluralité de particules respirables.

21. Procédé d'utilisation selon la revendication 19, dans lequel l'étape consistant à produire une pression de fluide comprend l'étape consistant à :
produire un écoulement de fluide adjacent à la dose concentrée à partir du fluide qui est initialement généralement exempt de toute vitesse, de sorte que l'écoulement de fluide atteint par conséquent une vitesse maximale rapidement après que le fluide a commencé de s'écouler.

22. Procédé d'utilisation selon la revendication 19, dans lequel l'étape consistant à produire une pression de fluide comprend l'étape consistant à :
produire un écoulement de fluide qui se transforme en un écoulement de fluide turbulent et très rapide par rapport à la dose concentrée, qui désintègre la dose concentrée en une pluralité de particules respirables.

23. Procédé d'utilisation selon la revendication 22, dans lequel l'écoulement de fluide turbulent et très rapide est fourni rapidement après que le fluide a commencé de s'écouler.

24. Procédé d'utilisation selon la revendication 19, dans lequel lesdites étapes consistant à fournir des particules micronisées du médicament (12) à l'intérieur d'une enveloppe (14) et consistant à fournir les particules micronisées sous la forme d'une dose agglomérée prédéterminée dans des moyens de chambre (32) comprend les étapes consistant à :
prévoir un organe de dosage (30) dont les surfaces définissent les moyens de chambre (32), dans lequel les moyens de chambre (32) ont un axe longitudinal (f) et s'étendent à travers l'organe de dosage (30) entre des parties espacées d'une surface jointive extérieure (33) ; et
conserver une quantité de médicament en poudre sèche (12) dans un réservoir à médicament (11) ;
utiliser un dispositif d'agglomération situé à l'intérieur du réservoir à médicament (11) qui fournit une force de compactage positive, indépendante de l'orientation qui permet de charger ledit médicament en poudre sèche (12) à partir du réservoir à médicament (11) dans les moyens de chambre (32) sous une pression suffisante pour concentrer le médicament en poudre sèche et en faire une dose reproductible.

25. Procédé d'utilisation selon la revendication 24, dans lequel le dispositif d'agglomération se déplace à travers les moyens de chambre (32) si bien que la force de compactage positive, indépendante de l'orientation a un composant qui est généralement parallèle à l'axe longitudinal (f).

26. Procédé d'utilisation selon la revendication 25, comprenant en outre l'étape consistant à augmenter progressivement le composant de la force de compactage qui est généralement parallèle à l'axe (f) à mesure que le dispositif d'agglomération se déplace à travers au moins une partie des moyens de chambre (32).

27. Procédé d'utilisation selon la revendication 19, dans lequel l'étape consistant à produire une pression de fluide comprend l'étape consistant à injecter une pression de fluide généralement immédiatement adjacente à la dose.

28. Procédé d'utilisation selon la revendication 19, dans lequel l'étape consistant à produire une pression de fluide comprend les étapes consistant à :
produire une première pression qui est plus élevée que la pression ambiante ;
positionner la dose adjacente à la pression ambiante ;
injecter la première pression afin de fournir une pression différentielle à travers la dose concentrée de sorte que le fluide s'écoule et vienne désintégrer la dose ; et
dans lequel une pression différentielle fournie à travers la dose correspond à la pression différentielle maximale susceptible d'être produite avec la première pression et la pression ambiante.
